(19) Europäisches Patentamt
European Patent Office
Office européen des brevets

(11) **EP 2 258 268 B1**

(12) **EUROPEAN PATENT SPECIFICATION**

(45) Date of publication and mention
of the grant of the patent:
**05.11.2014 Bulletin 2014/45**

(51) Int Cl.:
**G03B 42/04** (2006.01)   **A61B 6/00** (2006.01)
**G01T 1/20** (2006.01)

(21) Application number: **09727435.1**

(22) Date of filing: **20.02.2009**

(86) International application number:
**PCT/JP2009/053006**

(87) International publication number:
**WO 2009/122808 (08.10.2009 Gazette 2009/41)**

(54) **Cassette type radiation image detector**

Kassettenförmiger Strahlungsbilddetektor

Détecteur d'image de rayonnement de type cassette

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **31.03.2008 JP 2008090923**

(43) Date of publication of application:
**08.12.2010 Bulletin 2010/49**

(60) Divisional application:
**14185974.4**

(73) Proprietor: **Konica Minolta Medical & Graphic, Inc. Tokyo 191-8511 (JP)**

(72) Inventors:
• **AOYAGI, Shigeru**
**Hino-shi**
**Tokyo 191-8511 (JP)**
• **SUMI, Makoto**
**Hino-shi**
**Tokyo 191-8511 (JP)**

(74) Representative: **Gille Hrabal**
**Brucknerstrasse 20**
**40593 Düsseldorf (DE)**

(56) References cited:
WO-A1-2009/054242    JP-A- 6 019 015
JP-A- 2005 275 011    JP-A- 2006 087 566
US-A- 4 021 668    US-A- 4 638 501
US-A- 4 841 558    US-B2- 7 189 972

EP 2 258 268 B1

## Description

## FIELD OF THE INVENTION

The present invention relates to a cassette type radiation image detector.

## TECHNICAL BACKGROUND

Conventionally, a radiation image captured by using radiation, represented by X rays, has been widely employed for the purpose of diagnosing various kinds of diseases or the like. Although a screen film had been conventionally employed for capturing such the radiation image for medical use as abovementioned, in recent years, a trend of employing digitalized radiation images has been gradually proliferated in the market. For instance, there has been widely proliferated in the medical market such the CR (computed radiography) that acquires radiation image data, in such a manner that radiation penetrated through the subject (patient) are accumulated onto a photo-stimulable phosphor sheet on the surface of which photo-stimulable phosphor layer is formed, and then, the photo-electric conversion processing is applied to the phosphor light, emitted from the photo-stimulable phosphor sheet by scanning the concerned photo-stimulable phosphor sheet with a laser beam, so as to generate the radiation image data (for instance, set forth in Patent Documents 1 and 2, etc.)

Generally speaking, a cassette that accommodates a recording medium, such as a screen film, a photo-stimulable phosphor sheet, etc., therein, (for instance, refer to Patent Documents 1 and 2, etc.) is employed for the radiation image capturing operation. In this connection, the CR use cassette, to be used for the image capturing operation conducted by employing the CR, is designed and manufactured in conformity with the dimensional sizes specified in the JIS (Japan Industrial Standard) or the IEC (International Electro-technical Commission), so that the existing installations, such as a cassette holder, a bucky table, etc., which have been installed on the premise that those are in conformity with the conventional screen-film use cassette, are continuously available. In other words, in the conventional system, the compatibility of the dimensional sizes of the cassette concerned is well maintained, so as to achieve the effective utilization of the medical facilities and the digitalization of the radiation image data.

Further, in recent years, as a tool for acquiring a radiation image for medical use, there has been well known a Flat Panel Detector (hereinafter, referred to as a FPD, for simplicity) that serves as a detector for detecting radiation irradiated onto the subject and penetrated therethrough so as to acquire digital radiation image data (for instance, refer to Patent Documents 3, etc.).

Still further, a portable type image capturing apparatus that accommodates the abovementioned FPD inside the housing (portable type FPD) has been brought into practical use. It is expected that such the portable type detector as abovementioned will be widely employed for various kinds of medical activities, since the portability of the concerned detector makes it possible not only to conduct the radiation image capturing operation in the patient's bedroom or the like, but also to freely adjust its position and angle corresponding to the position and angle of the portion to be captured.

Patent Document 1: JP 2005-121783 A

Patent Document 2: JP 2005-114944 A

Patent Document 3: JP 9-73144 A

Patent Document 4: JP 2002-31152 A

Patent Document 5: US 7,189,972 B2

## DISCLOSURE OF THE INVENTION

## SUBJECT TO BE SOLVED BY THE INVENTION

As aforementioned, the dimensional sizes of the CR use cassette, currently in practical use, are in conformity with the dimensional sizes of the screen/film use cassette, specified in the JIS or the IEC, and other devices, such as a bucky table, etc., are also manufactured in conformity with the dimensional sizes specified in the JIS or the IEC. Accordingly, with respect to the FPD abovementioned, if the FPD could be used in such a state that the concerned FPD is accommodated into a cassette in conformity with the dimensional sizes specified by any one of the abovementioned standards, it would become possible to utilize the existing installations, currently installed in the facility concerned, for the radiation image capturing operation employing the FPD, and as a result, it would become possible to suppress an amount of facility investment, being necessary for introducing the FPD as a radiation image capturing tool, at a minimum level.

However, the shape of the portable and cassette type radiation image detector (portable type FPD), which is accommodated into the conventional housing as set forth in Patent Documents 4 and 5, is not in conformity with that specified in the JIS or the IEC, and accordingly, is unavailable in any one of the existing installations.

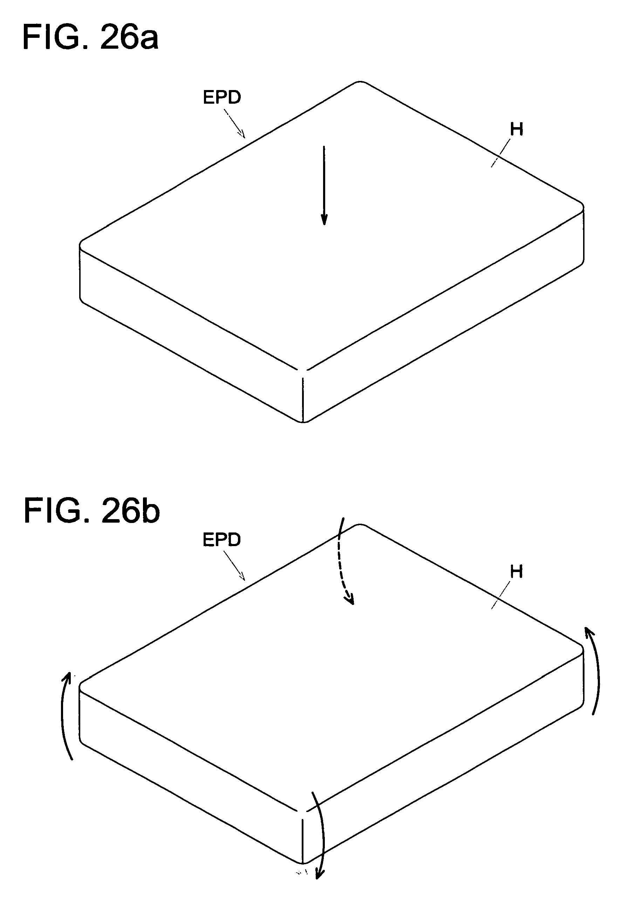

On the other hand, if the cassette type radiation image detector were formed according to the dimensional sizes specified in the JIS or the IEC, its outer shape would become a thin plate as a whole. As a result, compared to such the case that a housing H of a cassette type radiation image detector FPD is very thick, the housing H, shaped in the thin plate, is liable to suffer from not only the bending distortion as shown in Fig. 26a, but also the twisting distortion as shown in Fig. 26b.

Specifically, when the body weight of the patient (load) is applied onto the cassette type radiation image detector, the bending distortion and the twisting distortion, as abovementioned, are generated onto its housing. Accordingly, it is necessery to improve the body strength of the cassette type radiation image detector as a whole, to such an extent that the distortions of the housing can be sufficiently suppressed, so that the load is not applied to the various kinds of vulnerable parts mounted therein, such as a glass substrate, electronic parts, etc., even if such the load as abovementioned is applied to the cassette type radiation image detector concerned.

Accordingly, to solve the abovementioned problems, the present invention has been achieved. Concretely speaking, the object of the present invention is to provide a cassette type radiation image detector, which serves as an FPD (Flat Panel Detector) that makes it possible to achieve the digitalization of the image data, and which is shaped in a thin-type detector having compatibility with the CR use cassette and bas such a robust strength that is sufficient for suppressing the deformation of the housing against an external force, and which is also makes it possible to conduct a portable image capturing operation.

## MEANS FOR SOLVING THE SUBJECT

To achieve the abovementioned objects of the present invention, a radiation image detector according to claims 1-7 is provided.

## EFFECT OF THE INVENTION

According to the cassette-type radiation image detector embodied in the present invention, since the main section of the housing is formed by employing the plate-shape carbon fibers, in such a manner that the direction of carbon fibers, included in the radiation incident surface, and the other direction of carbon fibers, included in the opposite surface of it, are inclined relative to the opening sections, respectively, and are different from each other, even if the cassette type detector 1 is made to be a thin-type detector and would be formed in a thin plate shape as a whole, it becomes possible to sufficiently and appropriately suppress not only the bending deformation of the housing as shown in Fig. 26a, but also the twisting deformation to be generated by the tension applied in an inclined direction with respect to both the long side and the short side of the housing as shown in Fig. 26b. As a result, it becomes possible to achieve the sufficient strength being necessary for the full weight (body weight) image capturing operation in which the body weight of the patient is applied onto the cassette concerned.

Accordingly, it becomes possible to form the cassette type radiation image detector, which serves as an FPD (Flat Panel Detector) that makes it possible to achieve the digitalization of the image data, in shape of a thin-type detector having compatibility with the CR use cassette. Further, since the cassette type radiation image detector, embodied in the present invention, has such a robust strength that is sufficient for suppressing the deformation of the housing against an external force, it becomes possible to suppress the deformation of the sensor panel incorporated inside the housing, and as a result, a malfuntion of the sensor panel can be prevented. Still further, the cassette type radiation image detector abovementioned is portable for the user, so as to make it possible to conduct an image capturing operation in a portable mode.

## BRIEF DESCRIPTION OF THE DRAWINGS

Fig. 1 shows a perspective view of a cassette type radiation image detector embodied in the present invention.

Fig. 2 shows an exploded perspective view of a housing embodied in the present invention.

Fig. 3a shows a cross sectional schematic diagram indicating a buffering member of a second covering member, a cross sectional form of which is shaped in substantially an alphabetical letter "V", Fig. 3b shows a cross sectional schematic diagram indicating a state that a detection unit is guided and moved to a horizontal position, and Fig. 3c shows a cross sectional schematic diagram indicating a state that a detection unit is supported by a buffering member.

Fig. 4 shows a schematic diagram indicating a rough internal configuration of a cassette type detector shown in Fig. 1.

Fig. 5 shows a cross sectional schematic diagram indicating a rough internal configuration of a cassette type detector cut along A - A line shown in Fig. 4.

Fig. 6 shows a cross sectional schematic diagram indicating a rough internal configuration of a cassette type detector cut along the cutting line of "A - A" shown in Fig. 4.

Fig. 7 shows a schematic diagram indicating a plane view of a detection panel.

Fig. 8 shows a cross sectional schematic diagram indicating a side view of a detection panel, when viewing it in a direction of arrow F.

Fig. 9 shows a cross sectional schematic diagram indicating a detection panel cut along the cutting line of "G - G" shown in Fig. 7.

Fig. 10 shows a graph indicating loads to be applied onto a glass substrate in various kinds of image capturing postures.

Fig. 11 shows a schematic diagram indicating a rough configuration of a pressure measuring apparatus.

Fig. 12 shows a table indicating allowable stresses of various kinds of glass substrates.

Fig. 13 shows a table indicating a maximum stress and a maximum amount of bending deformation to be generated in a glass substrate, when four sides of the glass substrate are supported.

Fig. 14 shows an explanatory schematic diagram indicating a glass substrate, when four sides of the glass substrate are supported.

Fig. 15 shows a table indicating coefficients in a glass substrate, when four sides of the glass substrate are supported.

Fig. 16 shows an equivalent electronic circuit diagram indicating a photoelectric converting section in regard to one of pixels constituting a signal detecting section.

Fig. 17 shows an equivalent electronic circuit diagram indicating a configuration in which a plurality of photoelectric converting sections, each shown in Fig. 16, are aligned in a two dimensional pattern.

Fig. 18 shows a schematic diagram indicating a plate-shaped carbon fibers, which forms a housing main section.

Fig. 19 shows a table indicating elasticity and thermal conductivity of each of various kinds of materials for comparison purpose.

Fig. 20 shows an explanatory schematic diagram for explaining that directions of carbon fibers on a radiation incident surface of a housing main section and its opposite surface are different from each other.

Fig. 21 shows a graph indicating simulation results in regard to bending deformation of a housing.

Fig 22a shows an explanatory schematic diagram indicating a distribution of a load when only one end portion of a cassette type detector is supported in a manner like cantilever. While, Fig 22b shows an explanatory schematic diagram indicating a distribution of a load when two ends of the cassette type detector, which are respectively positioned at diagonal opposite angles, are supported.

Fig. 23a shows a schematic diagram indicating a plate-shaped carbon fibers in which a rolling direction is established as an extended direction of a short side of a housing. While, Fig. 23b shows a schematic diagram indicating a plate-shaped carbon fibers in which a rolling direction is established as an extended direction of a long side of a housing.

Fig. 24 shows a table indicating angles $\theta$ and angles $\theta^*$, which are applicable when a cassette type radiation image detector is formed with dimensional sizes in conformity with the JIS or the IEC standard.

Fig. 25 shows a schematic diagram indicating an irregularity (concavities and convexities) formed on a surface of a plate-shaped carbon fibers.

Fig. 26a shows an explanatory schematic diagram indicating a bending deformation to be generated onto the cassette type radiation image detector. While, Fig. 26b shows an explanatory schematic diagram indicating a twisting deformation to be generated onto the cassette type radiation image detector.

**EXPLANATION OF THE NOTATIONS**

| | |
|---|---|
| 1 | a cassette type detector (cassette type radiation image detector) |
| 2 | a detection unit |
| 3 | a housing |
| 31 | a housing main section |
| 31A | a plate-shaped carbon fibers |
| 32 | a first covering member |
| 33 | a second covering member, |
| 151 | a signal detecting section |
| 211 | a scintillation layer |
| 311 | an opening |
| 312 | an opening |
| 324 | a connecting extension |
| 334 | a connecting extension |
| C | an irregularity |
| F | a direction of carbon fibers |
| F* | a direction of carbon fibers |
| S | a rolling direction |
| X | a radiation incident surface |
| Y | an opposite surface |
| $\theta$ | angle |

**BEST MODE FOR IMPLEMENTING THE INVENTION**

Referring to the drawings, the cassette type radiation image detector, embodied in the present invention, will be detailed in the following. However, the scope of the present invention is not limited to the examples shown in the drawings.

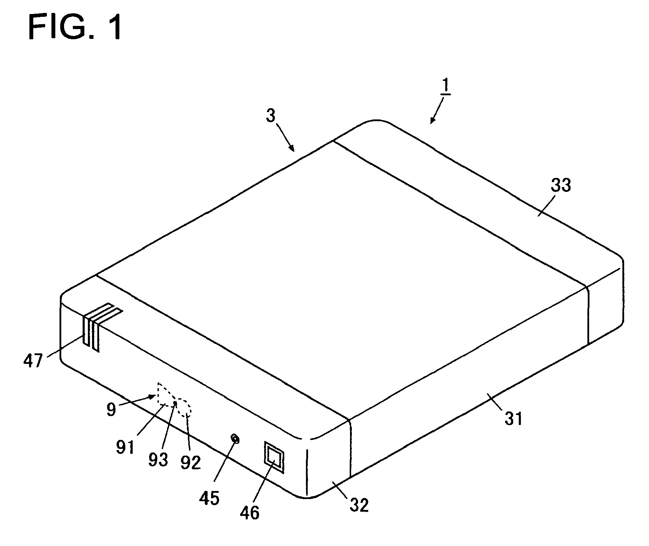

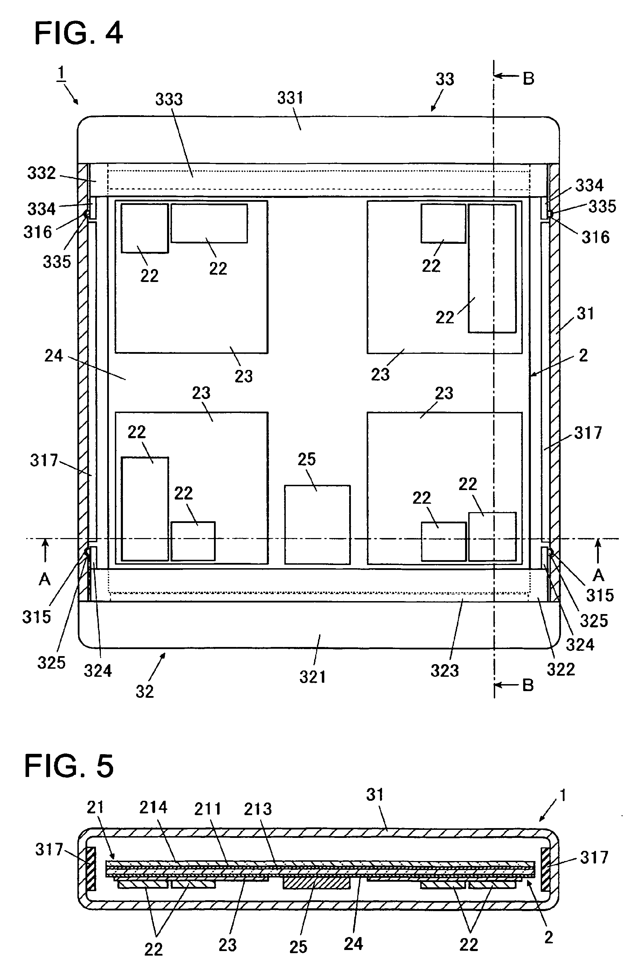

Fig. 1 shows a perspective view of the cassette type radiation image detector (hereinafter, referred to as the cassette type detector), embodied in the present invention. A cassette type detector 1, embodied in the present invention, is defined as a cassette type FPD (Flat Panel Detector), and provided with a detection unit 2 that detects the radiation irradiated and penetrated through the subject so as to acquire radiation image information as digital image data (refer to Fig. 4), and a housing 3 that accommodates the detection unit 2 therein.

In the present embodiment, the housing 3 is formed in such a shape that the thickness of the housing 3 in an incident direction of the radiation is equal to or smaller than 16 mm, so as to comply with the dimensional sizes, specified in the JIS (Japan Industrial Standard) Z 4905, which is specified in regard to the CR use cassette and the conventional screen/film use cassette aforementioned, and the IEC (International Electro-technical Commission) 60406, serving as the international standard corresponding to JIS Z 4905.

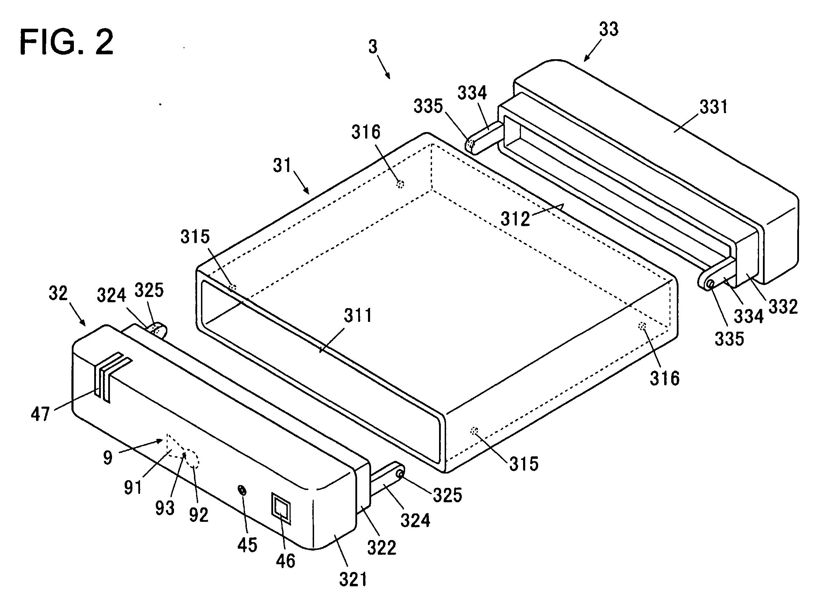

Fig. 2 shows an exploded perspective view of the housing 3, embodied in the present invention. As shown in Fig. 2, the housing 3 is provided with a housing main section 31 that is formed in a rectangular hollow cylinder having an opening 311 and another opening 312 at both end portions thereof, and a first covering member 32 and a second covering member 33 that cover the opening 311 and the other opening 312, respectively, so as to tightly seal both ends of the housing main section 31.

The first covering member 32 and the second covering member 33 are provided with a covering main section 321 and another covering main section 331, and an insertion section 322 and another insertion section 332, respectively, which are made of a non-electro-conductive material, for instance, a non-electro-conductive plastic, etc.

The covering main section 321 and the covering main section 331 are formed in such a shape that the sizes of outer circumferences of them are substantially equal to those of the opening 311 and the opening 312, respectively. The sizes of the covering main section 321 and the covering main section 331 in inserting directions towards the opening 311 and opening 312 are established at 8 mm, respectively. In this connection, although the limitations of the abovementioned sizes of the covering main section 321 and the covering main section 331 are not specified in the present embodiment, it is preferable that the size of the covering main section 321 to which an antenna device 9, detailed later, is mounted is established as such a value being equal to or greater than 6 mm, or more preferably, a value being equal to or greater than 8 mm.

Further, each of the insertion section 322 and the insertion section 332 is formed in a frame shape having an opening to be faced against corresponding one of the opening 311 and the opening 312. Still further, the insertion section 322 and the covering main section 331 are formed in such a shape that the sizes of outer circumferences of them are slightly smaller than the inner circumferences of the opening 311 and the opening 312, respectively.

Still further, a buffering member 323 and another buffering member 333 (refer to Fig. 4, etc.) for absorbing an external force transmitted into the detection unit 2 from an external environment are equipped inside the insertion section 322 and the insertion section 332, respectively. The scope of the material to be employed for the buffering member 323 and the buffering member 332 is not specifically limited, and, for instance, an urethane foam, a silicone material can be employed for this purpose.

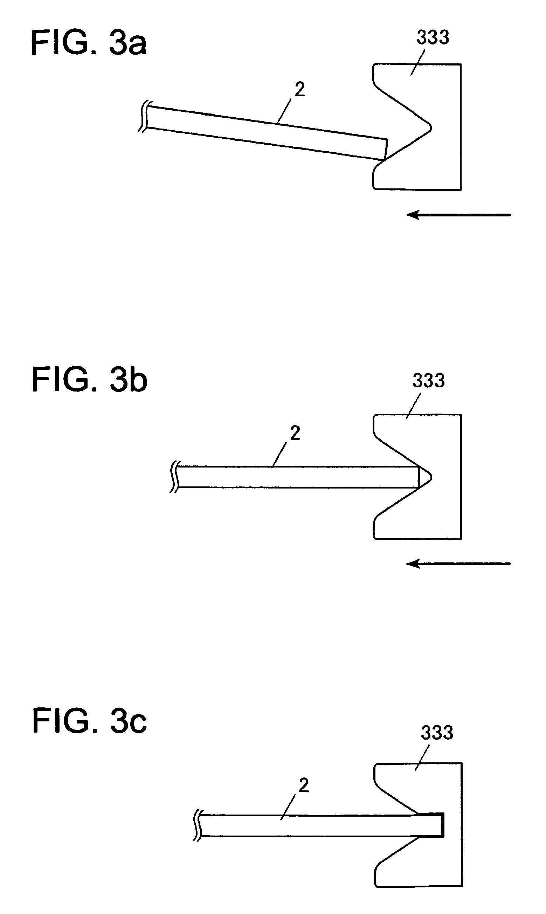

Specifically, the buffering member 333 to be equipped into the insertion section 332 is formed in such a shape that the cross section of the buffering member 333 is substantially shaped in an alphabetical letter "V", so that the end portion of the detection unit 2 is guided into a horizontal direction along the V-shaped inclination surface of the buffering member 333, as shown in Fig. 3a. Further, if the buffering member 333 is made of a kind of deformable material, such as an elastic material, a viscosity material, a visco-elastic material, or the like, when the second covering member 33 is pushed into the housing main section 31, the shape of the buffering member 333 would be deformed in accordance with the shape of the end portion of the detection unit 2, so as to tightly hold the end portion of the detection unit 2, as shown in Fig. 3b and Fig 3c. As abovementioned, the buffering member 333 also serves as a holding section for holding the detection unit 2 at an appropriate position within the housing 3.

As shown in Fig. 2, connecting extensions 324 and other connecting extensions 334, serving as connecting members for connecting the housing main section 31 with the first covering member 32 and the second covering member 33, are extended towards the inserting directions against the opening 311 and the opening 312, respectively. Further, engagement protrusions 325 and other engagement protrusions 335 are formed onto the outer surfaces of the connecting extensions 324 and the connecting extensions 334, respectively.

In this connection, it is preferable that the outer circumferential surfaces of the insertion section 322 and the insertion section 332 are equipped with ling-shaped waterproofing members (not shown in the drawings) made of an elastic rubber or the like, respectively. Since the adhesion property between the housing main section 31 and each of the first covering member 32 and the second covering member 33 can be increased by inserting the ling-shaped waterproofing member between them, it becomes possible to prevent various kinds of foreign matters, such as powder dust, sweat of patient, aqua component of antiseptic solution, etc., from entering into the inside of the housing 3.

The antenna device 9, to be used for bilaterally communicating information in a wireless communication mode between the cassette type detector 1 and an external apparatus, is embedded into a surface, which is one of side surfaces of the covering main section 321 of the first covering member 32 and which is orthogonal to the radiation incident surface of the cassette type detector 1.

The antenna device 9 is provided with a pair of radiation plates 91 and 92, each made of a metal plate and a driving electric power feeding section 93 to feed a driving electric power to the pair of radiation plates 91 and 92. In the present embodiment, the radiation plate 91, serving as a member of the pair of radiation plates 91 and 92, is formed in a trapezoidal shape as its front view, while, the radiation plate 92, serving as another member of the pair of radiation plates 91 and 92, is formed in substantially a circular shape as its front view. Further, the driving electric power feeding section 93 is coupled to substantially a center of the upper bottom section of the radiation plate 91, and also coupled to a part of the radiation plate 92. Since the radiation plate 91 and the radiation plate 92 are connected with each other while placing the driving electric power feeding section 93 between them, a predetermined gap is formed between the radiation plate 91 and the radiation plate 92.

In this connection, the scopes of the kind and the shape of the antenna device 9 applicable to the present invention are not limited to the abovementioned example. Further, the scope of the present invention is not limited to the abovementioned example in which the antenna device 9 is embedded into the covering main section 321, but it is also applicable that the antenna device 9 is adhered to the outer surface or the inner surface of the covering main section 321. However, since the receiving sensitivity and the receiving gain would be deteriorated when the antenna device 9 is disposed at such a position that is located near an electro-conductive member, made of an electro-conductive material, such as a metal, a carbon, etc., it is preferable that the antenna device 9 is disposed at a position being apart from the housing main section 31, which is formed by employing an electro-conductive material, such as a carbon, etc., and various kinds of electric parts 22 (refer to Fig. 4), which are formed by employing an electro-conductive material, such as a metal, etc., as far as possible. It is preferable that the separating distance between them is equal to or greater than 6 mm, and it is more preferable that the separating distance between them is equal to or greater than 8 mm.

In regard to the abovementioned point, the antenna device 9 employed in the present embodiment is embedded into the covering main section 321 made of a non-electro-conductive material as aforementioned, and the size of the covering main section 321 in the inserting direction for the opening 311 is set at 8 mm. Accordingly, the antenna device 9 is disposed at the position being 8 mm apart from the housing main section 31, which is formed by including an electro-conductive material, such as carbon fibers, etc., and as a result, the receiving sensitivity and the receiving gain can be preferably maintained.

Further, as shown in Fig. 1 and Fig. 2, on the surface same as one of the surfaces of the covering main section 321, into which the antenna device 9 is embedded, a charging terminal 45, to which an external power source or the like is to be coupled when a rechargeable buttery 25 (refer to Fig. 4), mounted inside the housing 3, is to be recharged, is formed, and, in addition, an electric power switch 46, for turning ON or OFF the electric power supply to be fed to the cassette type detector 1 from the electric power source, is disposed. Still further, an indicator 47, which is constituted by, for instance, a LED (Light Emitting Diode) or the like so as to indicate the charging status of the rechargeable battery 25, various kinds of operating statuses, etc., at the corner section formed by the surface into which the antenna device 9 is embedded and the other surface of radiation incident side.

In this connection, although all of the antenna device 9, the charging terminal 45, etc., are equipped onto the first covering member 32 in the present embodiment, it is also applicable that the cassette type detector 1 is so constituted that all of or a some of them are equipped onto the second covering member 33. Further, the scope of the interface use parts, such as the antenna device 9, the charging terminal 45, etc., is not limited to those exemplified in the present embodiment described in the foregoing, it is also applicable that any other part is included, or any one of the above-exemplified parts is excluded.

The configurations and operations of the housing main section 31 will be detailed later on.

As shown in Fig. 2 and Fig. 4, inside the housing main section 31, engagement dent sections 315 and 316, which are to be respectively engaged with the engagement protrusions 325 and 335, are formed at such positions that respectively correspond to the engagement protrusions 325 and 335 of the connecting extensions 324 and 334 of the first covering member 32 and second covering member 33.

The cassette type detector 1 is so constituted that both the opening 311 and the opening 312 of the housing 3 is tightly closed by inserting the insertion section 322 of the first covering member 32 into the opening 311 located at one end portion of housing main section 31, and inserting the insertion section 332 of the second covering member 33 into the opening 312 located at the other end portion of housing main section 31, and then, respectively engaging the engagement protrusions 325 and 335 with the engagement dent sections 315 and 316, so as to seal up the inside of the housing 3 and to integrate them into one body. In this connection, the scope of method and structure for connecting the housing main section 31 to the first covering member 32 and second covering member 33 is not limited to the above-exemplified embodiment. For instance, it is also applicable that either the screw fastening method or the adhesive fixing method is employed for this purpose.

Further, in this connection, the present embodiment is so constituted that the first covering member 32 and the second covering member 33 are firmly fixed onto the housing main section 31 after once the abovementioned assembling operation has been completed, so as to make it impossible to disassemble the cassette type detector 1 into the parts again. According to this feature, it becomes possible to heighten the sealing capability of the housing 3. Owing to the abovementioned structure, for instance, at the time when the rechargeable buttery 25 should be changed, it is necessary to distract the first covering member 32 and the second covering member 33 to disassemble the cassette type detector 1. However, since the first covering member 32 and the second covering member 33, which are made of a resin material or the like, are relatively inexpensive, it is possible to suppress the loss, to be generated by destructing the covering members abovementioned, at a lower level, while it is possible to take out the detection unit 2 in a reusable state.

Still further, buffering members 317 (refer to Fig. 4), for protecting the detection unit 2 from a certain mechanical failure caused by interference between the detection unit 2 and the inner wall surfaces of the housing main section 31, are disposed along both side sections inside the housing main section 31. In this connection, although the scope of the material to be employed for the buffering members 317 is not limited specifically, for instance, a resin material having an elastic property, such as a silicon, a polyurethane, etc., can be employed for this purpose.

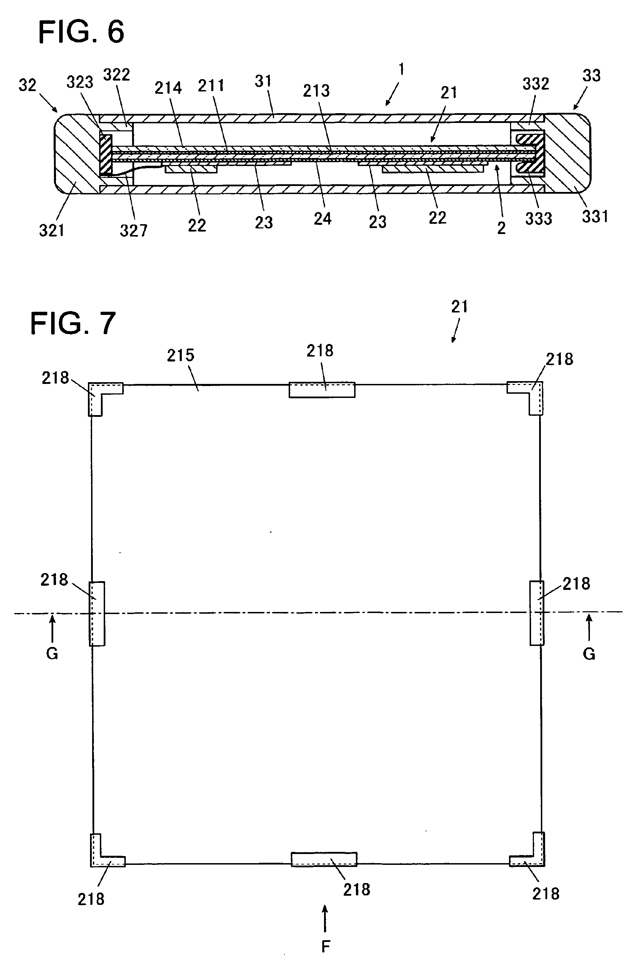

Fig. 4 shows a plane view indicating a state that the detection unit 2 is accommodated into the housing 3, viewing from upper side of the cassette type detector 1 (radiation incident side when capturing a radiation image). Fig. 5 shows a cross sectional schematic diagram along the cutting line of "A - A" shown in Fig. 4, while Fig. 6 shows another cross sectional schematic diagram along the cutting line of "B - B" shown in Fig. 4. In this connection, the schematic diagram shown in Fig. 4 schematically indicates an arrangement of various kinds of sectional members accommodated inside the cassette type detector 1, in such a state that the radiation incident side section and a detection panel 21, detailed later, are removed as a matter of convenience.

As shown in Fig. 4 through Fig. 6, the detection unit 2 is constituted by the detection panel 21, a circuit board 23 on which the various kinds of electric parts 22 are mounted, etc. In the present embodiment, the circuit board 23 is fixed onto a base plate 24 made of a resin material, etc., and by adhering the base plate 24 onto the detection panel 21, the circuit board 23 is fixed onto the detection panel 21 through the base plate 24. In this connection, since the base plate 24 is not such the element that is indispensable for the present invention, it is applicable that the circuit board 23, etc., are fixed directly onto the detection panel 21 without placing the base plate 24 between them.

As shown in Fig. 4, in the present embodiment, the circuit board 23 onto which the electric parts 22 are to be mounted is divided into four sections, each of which is disposed at a position near each of four corners of the detection panel 21. Further, the electric parts 22 are disposed at positions located along the outer circumferential surface of the detection panel 21, while mounting them onto the circuit board 23. It is preferable that each of the electric parts 22 is disposed at a position located near each of the four corners as nearer as possible. By disposing the electric parts 22 according to the abovementioned arrangement, when the detection unit 2 is accommodated into the housing 3, the electric parts 22 are aligned along the area of the peripheral edge portion of the housing main section 31, which is hardly deformed by an external force possibly applied to the housing 3 (high strength, or robust). In this connection, the scope of the factor, such as the number of sections acquired by dividing the circuit board 23, the number of the electric parts 22, the arranging method of them, etc., is not limited to those exemplified in the above.

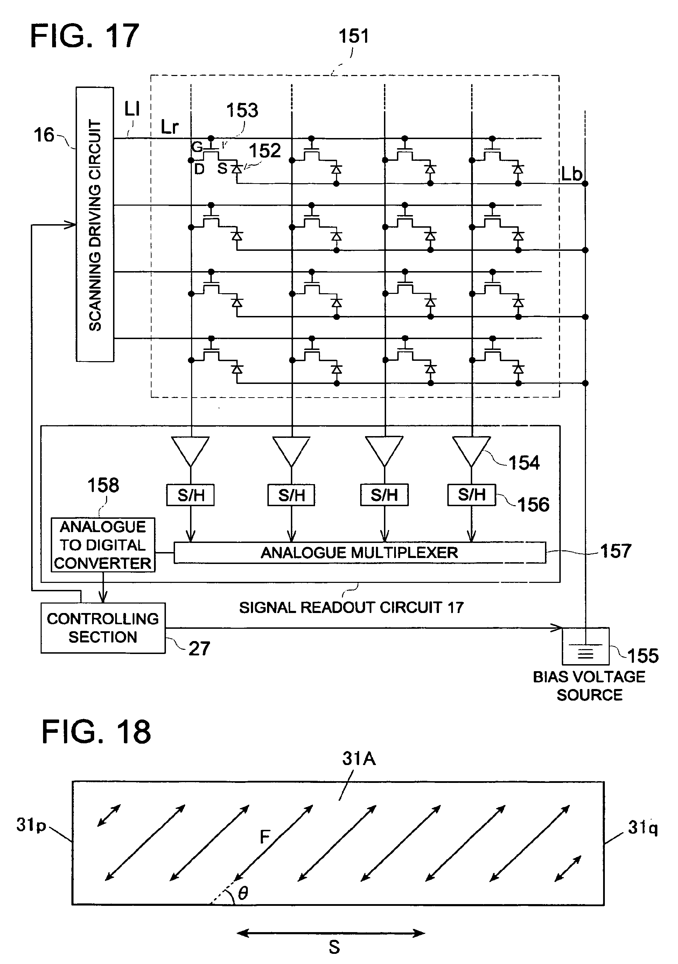

In the present embodiment, for instance, the electric parts 22 to be mounted onto the circuit board 23 include: a CPU (Central Processing Unit) constituting a controlling section 27 (refer to Fig. 17) to conduct various kinds of operations for controlling each of the sections; a storage section constituted by various kinds of storage devices, including a ROM (Read Only Memory), a RAM (Random Access Memory), etc., (hereinafter, not shown in the drawings); a scanning driving circuit 16 (refer to Fig. 17); a signal readout circuit 17 (refer to Fig. 17); etc. In this connection, it is also applicable that the cassette type detector 1 is provided with an image data storage section that is constituted by a rewritable exclusive-readout storage, such as a Flash Memory or the like, etc., other than the ROM and the RAM, so as to store image data outputted from the detection panel 21.

Further, the detection unit 2 is provided with a communication section (not shown in the drawings) to conduct operations for bilaterally communicating various kinds of data with an external device. For instance, the communication section is so constituted that the communication section transfers the image data outputted from the detection panel 21 to the external device through the antenna device 9 aforementioned, and receives various kinds of signals, such as a radiation-image capturing operation start signal, etc., transmitted from the external device through the antenna device 9.

Still further, at the position near the charging terminal 45, equipped on the first covering member 32, on the base plate 24 when the detection unit 2 is accommodated inside the housing 3, there is mounted the rechargeable buttery 25, serving as the electric power supplying section to supply electric power to various kinds of functional sections, including a plurality of driving sections that constitute the cassette type detector 1 (for instance, the scanning driving circuit 16, detailed later (refer to Fig. 17), the signal readout circuit 17 (refer to Fig. 17), the communication section, the storage section, a charge amount detecting section (not shown in the drawings), the indicator 47, the detection panel 21, etc.).

For instance, a freely rechargeable battery, such as a nickel-cadmium battery, a nickel metal hydride battery, a lithium ion battery, a small-sized seal lead battery, a lead storage battery, etc., can be employed as the rechargeable battery 25. Otherwise, it is also applicable that a fuel cell is employed for this purpose, instead of the rechargeable battery 25. In this connection, the scope of properties of the rechargeable battery 25, such as a shape, dimensional sizes, a number of cells to be incorporated, an arrangement, etc., is not limited to those exemplified in the schematic diagram shown in Fig. 4, etc.

By mounting the rechargeable battery 25 onto a predetermined position on the base plate 24, the rechargeable buttery 25 is automatically coupled to the charging terminal 45. Accordingly, for instance, by mounting the cassette type detector 1 onto a charging device, such as a cradle, etc., (not shown in the drawings) to be coupled to an external power source, the terminal of the changing device side is automatically coupled to the charging terminal 45, so as to implement the operation for charging the rechargeable buttery 25.

A flexible harness 327 made of an elastic material is disposed at the end portion of the circuit board 23 to which various kinds of electric parts 22 and the rechargeable buttery 25 are coupled. The circuit board 23, etc., are electrically coupled to the charging terminal 45, the electric power switch 46, the indicator 47, and the antenna device 9 through the flexible harness 327. In this connection, it is applicable that the flexible harness 327 is connected to the charging terminal 45 either by employing the connector, or by soldering them with each other.

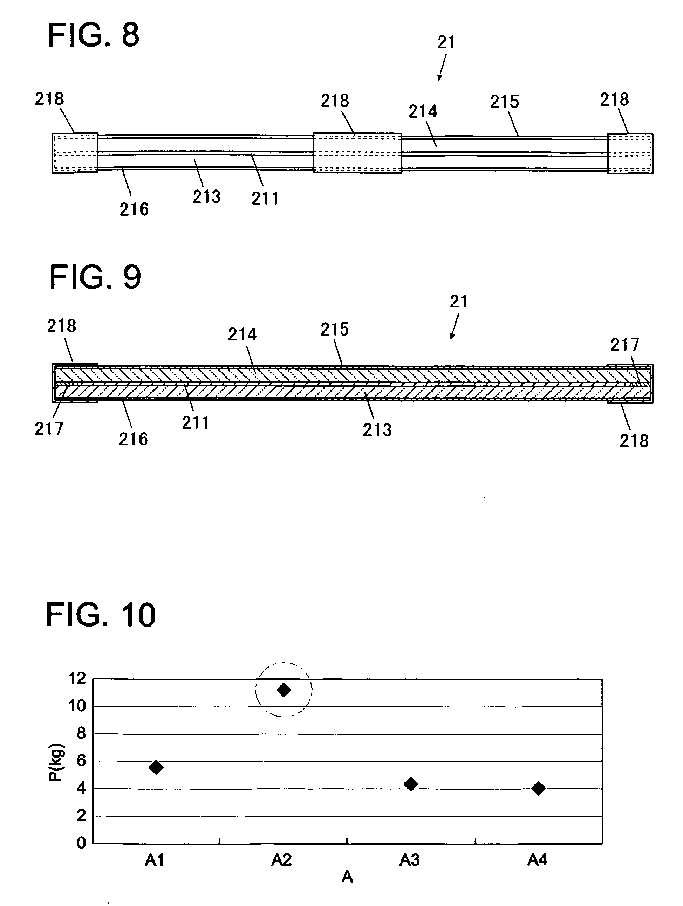

Fig. 7 shows a schematic diagram indicating a plane view of the detection panel 21; Fig. 8 shows a schematic diagram indicating a side view of the detection panel 21, when viewing it in a direction of arrow F; and Fig. 9 shows a schematic diagram indicating a cross sectional schematic diagram of the detection panel 21, along the cutting line of "G - G" shown in Fig. 7.

The detection panel 21 is constituted by: a first glass substrate 214 onto a surface of which a scintillation layer (light emitting layer) 211, serving as a scintillator to convert the incident radiation to light, is formed; a second glass substrate 213 onto another surface of which a signal detecting section 151 (refer to Fig. 17) that is laminated below the scintillation layer 211 to detect the light, converted from the radiation by the scintillation layer 211, so as to convert the detected light to electric signals, is formed; etc., and is configured as a laminated structure in which the abovementioned layers are laminated.

For instance, the scintillation layer 211 includes a fluorescent substance as its main gradient, so as to output (emit) an electro-magnetic wave, the wavelengh of which is in a range of 300 - 800 nm, namely, the electro-magnetic wave (light) representing a light from a violet color light to an infreared light in center of visible light, based on the incident radiation.

For instance, either a material including $CaWO_4$, etc. as its base gradient, or another material in which a light emitting main substance is actively added into its base gradient, such as $CsI:T1$, $Cd_2O_2S:Tb$, $ZnS:Ag$, etc., can be employed as the fluorescent substance to be employed for the scintillation layer 211. Further, when a rare-earth element is represented by symbol "M", the fluorescent substance, which is represented by the general formula of $(Gd, M, Eu)_2O_3$, can be employed for the scintillation layer 211. Specifically, the $CsI:T1$ and the $Cd_2O_2S:Tb$ are preferably employed to acquire a high quality image having a little noise, since the radiation absorbing rate and the light emitting efficiency of them are high.

The scintillation layer 211 is such a layer in which the fluorescent substance is coated onto the substrate (not shown in the drawings) made of one of various kinds of high molecular (polymer) matters, such as, a cellulose acetate film, a polyester film, a polyethylene terephthalate film, etc., in a shape of a laminated layer by employing, for instance, a vapor phase epitaxy method, and its fluorescent substance layer is constituted by columnar crystals of fluorescent substance. As the vapor phase epitaxy method, any one of a vapor deposition method, a sputtering method, a CVD (Chemical Vapor Deposition) method, etc., is preferably employed. Even in any one of the abovementioned methods, it is possible to epitaxially growth the fluorescent substance layer as the slender columnar crystals, being independent from each other, on the substrate.

Further, the scintillation layer 211 is adhered to the lower side surface of the first glass substrate 214 (surface opposite to the radiation incident surface at the time of image capturing operation), and a first glass protection film 215 is further laminated onto the upper side surface of the first glass substrate 214 (radiation incident surface at the time of image capturing operation). Still further, the second glass substrate 213 is further laminated onto the lower side surface of scintillation layer 211 (surface opposite to the radiation incident surface at the time of image capturing operation), and a second glass protection film 216 is further laminated onto the lower side surface of the second glass substrate 213.

The thickness of each of the first glass substrate 214 and the second glass substrate 213 is set at 0.6 mm for practical use. In this connection, the thickness of each of the first glass substrate 214 and the second glass substrate 213 is not limited to 0.6 mm. Further, it is also applicable that the thicknesses of the first glass substrate 214 and the second glass substrate 213 are different from each other.

Now, the results of the experiments conducted by the present invention to investigate a limit of the bending amount, which causes the failure of the first glass substrate 214 or the second glass substrate 213, constituting the detection panel 21, will be detailed in the following.

Fig. 1 shows a graph indicating results of measuring forces (stress) to be exerted onto the second glass substrate 213 and the first glass substrate 214 of the detection unit 2 by using a pressure measuring apparatus 7, when the radiation image capturing operation is conducted in such a state that the cassette type detector 1 is placed onto a material having relatively higher hardness, such as a bucky table, etc.

The housing main section 31 is formed by employing the PITCH carbon fibers having a tensile modulus of elasticity of 790 Gpa, and the cassette type detector 1, in which the height of side portion of the housing 3 is set at 16 mm and the thickness (plate thickness) of the housing main section 31 is set at 2 mm, is employed. Further, the dimensional sizes of the cassette type detector 1 are set at those of the half cut size (14 inch x 17 inch), which yield the bending deformation most easily.

In this connection, the load to be applied onto the cassette type detector 1 varies depending on the image capturing posture of the subject. Within an imaginable usage environment of the cassette type detector 1, the image capturing posture of the subject, which exerts a maximum load onto the cassette type detector 1, would occur in such a case that the patient lies on his side in a recumbent position on a bed and the cassette type detector 1 is placed between the buttock of the patient and the bed. The following measurements were conducted under the abovementioned condition.

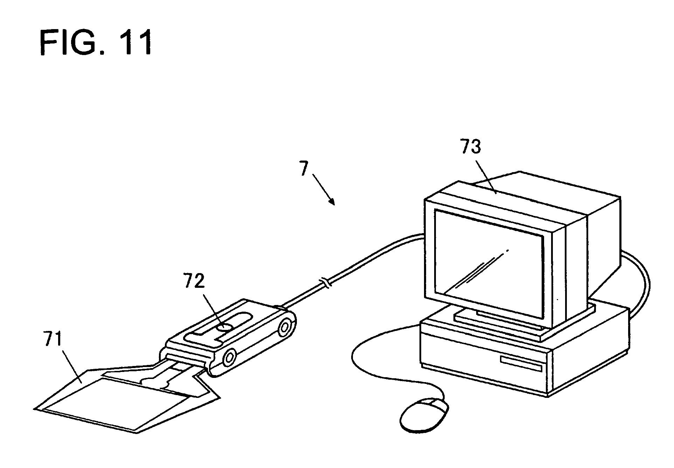

For instance, as shown in Fig. 11, the pressure measuring apparatus 7 is constituted by: a sensor sheet 71 that has a pressure sensitive element to convert the changes of the external applied pressure to electric signals and output the electric signals converted; and a computer 73 that receives the electric signals outputted by the sensor sheet 71. Concretely speaking, the I-SCAN system, manufactured by Nitta Co. Ltd. was employed for the measurements described in the following.

The measurements were conducted with respect to the four kinds of combinations of the image capturing posture of the patient and the image capturing part of the patient, including (A1) the cassette type detector 1 is placed below the buttock of the patient in the state that the patient lies on his back in a recumbent position, (A3) the cassette type detector 1 is placed below the back of the patient in the state that the patient lies on his back in a recumbent position, (A2) the cassette type detector 1 is placed below the buttock of the patient in the state that the patient lies on his side in a recumbent position, (A4) the cassette type detector 1 is placed below the shoulder of the patient in the state that the patient lies on his side in a recumbent position. Then, with respect to each of the combinations abovementioned, the sensor sheet 71 of the pressure measuring apparatus 7 is placed below the corresponding image capturing part of the patient, to measure the pressure applied onto the housing 3 of the cassette type detector 1.

As aforementioned, the image capturing posture of the patient, which exerts a maximum load onto the cassette type detector 1, would occur in such the case that the patient lies on his side in a recumbent position on a bed and the cassette type detector 1 is placed between the buttock of the patient and the bed. For instance, when the image capturing operation of the patient whose body weight is 100 kg is conducted, the maximum load to be exerted onto the second glass substrate 213 and the first glass substrate 214 of the detection unit 2 of the cassette type detector 1 is around 11 kg.

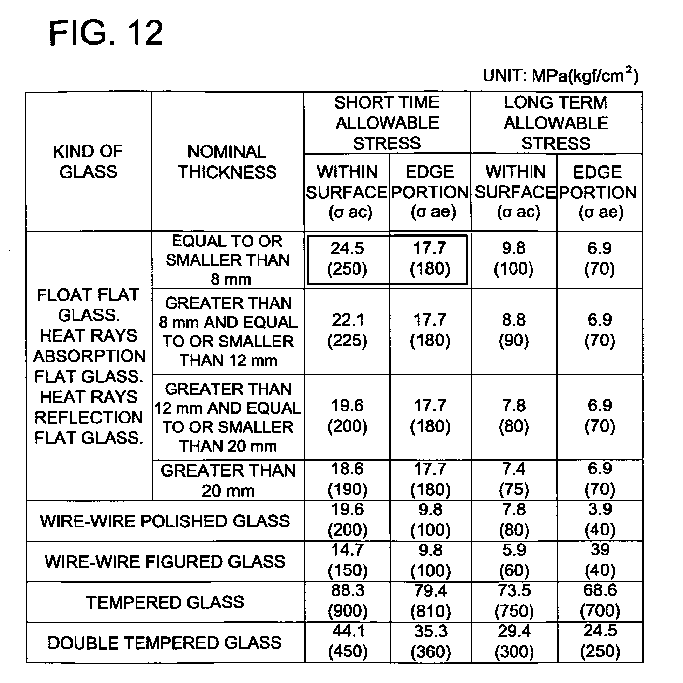

On the other hand, as shown in Fig. 12, when a glass plate (glass substrate) having a thickness equal to or smaller than 8 mm is employed, an allowable stress (short time allowable stress), to be generated against the force instantaneously exerted onto the glass substrate when the cassette type detector 1 is moved, is estimated as 24.5 MPa within the surface of the glass substrate concerned. Further, when a uniformly distributed load is applied onto a rectangular plate member in such a state that its four sides are supported as the second glass substrate 213 and the first glass substrate 214, the maximum stress is estimated as 23 MPa, while the maximum amount of bending deformation is estimated as 6 mm.

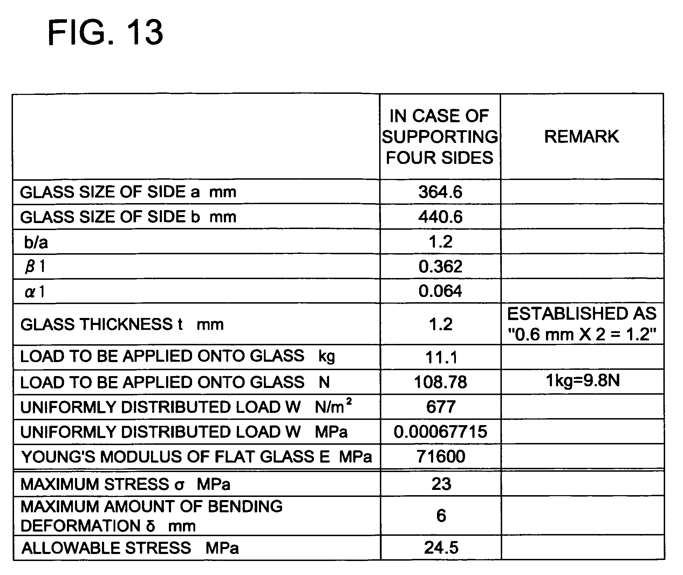

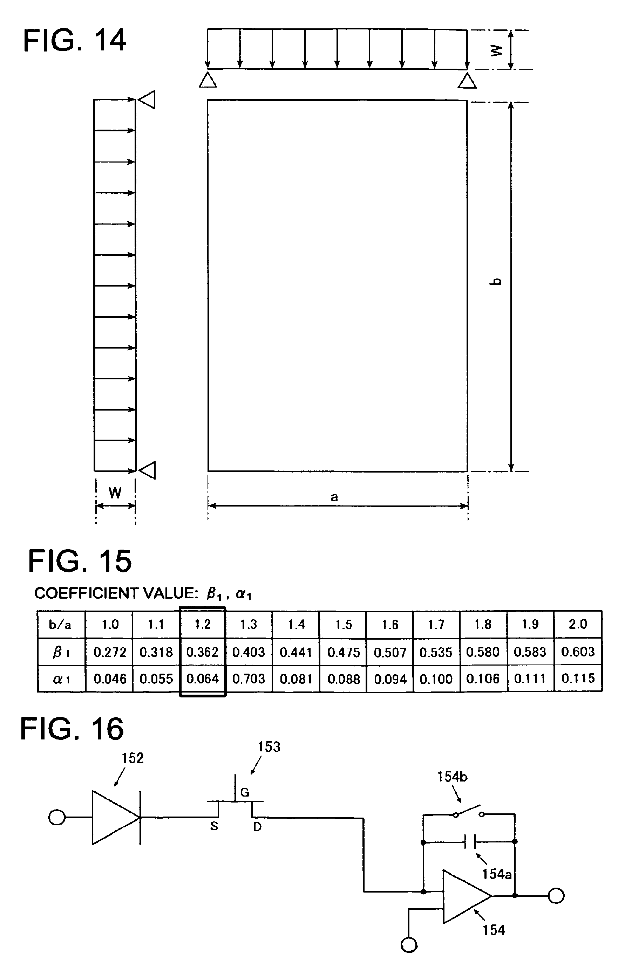

In this connection, when the length of the rectangular plate member, the four sides of which are supported, in its longitudinal direction is denoted by "b" and the other length of the rectangular plate member in the direction orthogonal to its longitudinal direction is denoted by "a" as shown in Fig. 14, the maximum stress denoted by "σ" shown in Fig. 13 is calculated by employing Equation (1), shown as follow, after the coefficients α1 and β1, corresponding to the case that the side length ratio of "b/a" is 1.2, is determined (refer to Fig. 15). Further, the maximum amount of bending deformation denoted by "δ" shown in Fig. 13 is calculated by employing Equation (2), also shown as follow, under the condition same as the above.

$$\sigma = \beta_1 \frac{W \cdot a^2}{t^2} \quad \cdots (1)$$

$$\delta = \alpha_1 \frac{W \cdot a^4}{E \cdot t^3} \quad \cdots (2)$$

As abovementioned, the maximum stress, generated at the time when the maximum load being assumable in the case of using the cassette type detector 1 is applied, is estimated as 23 MPa, while the maximum amount of bending deformation under the same condition, is estimated as 6 mm. In contrast to the above, as the first glass substrate 214 and the second glass substrate 213, employed in the present embodiment, the allowable stress of the glass plate (glass substrate) having a thickness equal to or smaller than 8 mm (practically, around 0.6 mm) is at least 24.5 MPa and a bending deformation within a range equal to or smaller than 6 mm is allowed.

As abovementioned, since the allowable amount of bending deformation of detection unit 2 is 6 mm, when conducting the full weight image capturing operation in which all of the patient's weight is applied onto the housing 3, even if the operator moves the cassette type detector 1 which has been once set under the patient, changes its position, etc., each of the maximum stresses generated in the first glass substrate 214 and the second glass substrate 213 does not exceed the allowable stress of the first glass substrate 214 and the second glass substrate 213, and as a result, does not induce a failure of the first glass substrate 214 and the second glass substrate 213, such as a crack, etc.

Further, by employing the laser beam to cut the edge surfaces of the first glass substrate 214 and the second glass substrate 213 (refer to Fig. 8), a smooth processing is applied to the edge surfaces, namely the cut surfaces, the ridge lines formed by the cut surfaces and the upper surfaces of the glass substrates, and the other ridge lines formed by the cut surfaces and the lower surfaces of the glass substrates.

In this connection, the smooth processing, which is to be conducted by employing the laser beam to cut the edge surfaces of the first glass substrate 214 and the second glass substrate 213, will be detailed in the following.

Generally speaking, the glass cutting operation is achieved by passing through two working processes including: initially, forming a streak (scar) on the glass plate by using a hard and acute tool so as to form a vertical clack in its thickness direction (scribing operation); and applying a stress in such a manner that the vertical clack is made to expand, so as to cut the glass plate along the streak (cutting work). Further, in the abovementioned conventional method, the work for making the surface of the glass plate to be scarred (scribing operation) is conducted by employing any one of an ultra hard metal, an electro-deposition diamond and a sintering diamond, etc. However, there has been such a problem that, when a cutting tool made of any one of the ultra hard metal, the diamond, etc., abovementioned, is employed for making the surface of the glass plate to be scarred, microscopic irregularities (concavity and convexity) are generated on the cut (segmented) edge surface of the glass plate, which make the glass plate easily broken when a bending load or the like is applied onto the glass plate concerned, due to the stress concentration to be generated onto each of the microscopic irregularities.

On the other hand, in the present embodiment, the works for making the surfaces of the first glass substrate 214 and the second glass substrate 213 to be scarred (scribing operation) are conducted by employing the laser beam. When the laser beam is employed for the cutting works as abovementioned, since the cut (segmented) edge surfaces of the glass plate are smoothed, it becomes possible to increase the strength of the glass plate against the bending load or the like.

Since the breakage of the glass substrate is caused by the fact that burs and/or irregularities, at each of which the stress concentration is liable to occur at the time of cutting the glass substrate, are partially formed on the cut edge surface of the glass substrate, instead of caused by the strength of the external force to be applied onto the glass substrate, by applying the smooth processing to the cut edge surface of the glass substrate as abovementioned, it becomes possible to prevent the breakage of the glass substrate even if an external force having considerable strength (stress) is applied onto the glass substrate concerned.

In this connection, as the cutting apparatus that employs the laser beam for cutting the edge surfaces of the first glass substrate 214 and the second glass substrate 213, for instance, the YAG (Yttrium Aluminum Garnet) laser apparatus that employs the YAG crystal as the laser optical medium at the laser oscillating section is preferably used. However, the scope of the cutting apparatus applicable in the present invention is not limited to the above.

A signal detecting section 151 (refer to Fig. 17), serving as a detection section that converts the electro-magnetic wave (light) outputted from the scintillation layer 211 to an electric energy so as to store the converted electric energy therein, and then outputs an image signal based on the electric energy stored, is formed on the upper side of the second glass substrate 213 (opposing to the scintillation layer 211 aforementioned).

As abovementioned, in the present embodiment, the signal detecting section 151 is laminated below the scintillation layer 211, so that the signal detecting section 151 and the scintillation layer 211 are closed into a space located between the second glass substrate 213, which is disposed below the signal detecting section 151, and the first glass substrate 214, which is disposed at the upper side of the scintillation layer 211, in such a state that the signal detecting section 151 and the scintillation layer 211 are opposing with each other in the closed space between the first glass substrate 214 and the second glass substrate 213.

Conventionally, there has been considered that it is impossible to prevent the breakage of the glass substrate, unless the stress to be exerted onto the glass substrate through the housing should be suppressed. Accordingly, a certain space has been provided between the housing and the glass substrate, and, in addition, various kinds of buffering members for alleviating the external force to be applied to the space concerned have been frequently employed, resulting in increase of outer dimensions of the housing concerned (large-sized housing).

In regard to this pint, the present inventors have found that the breakage of the glass substrate is originated from the fact that burs and/or irregularities, at each of which the stress concentration is liable to occur at the time of cutting the glass substrate, are partially formed, instead of occurs depending on the strength of the external forth to be exerted onto the glass substrate concerned. Accordingly, in order to eliminate the abovementioned burs and/or irregularities, which are liable to cause the stress concentration at the time of cutting the glass substrate, the present inventors have found it effective to apply the abovementioned smooth processing to the edge surface of the glass substrate. According to this smooth processing, it becomes possible to prevent the second glass substrate 213 and first glass substrate 214 from generating breakage of the glass substrate against the load and the bending deformation caused by the body weight of the patient, which is to be exerted onto the housing 3 having the aforementioned structure.

Further, a sealing member 217 is inserted between the first glass substrate 214 and the second glass substrate 213 along the outer circumferential edge lines of them, so as to make the first glass substrate 214 and the second glass substrate 213 adhere and connect with each other. According to this structure abovementioned, it becomes possible to increase the strength of the structure against the various kinds of loads, such as a bending moment, etc., stronger than ever.

Still further, when adhering the first glass substrate 214 and the second glass substrate 213 to each other, the deaerating operation is conducted by expelling air from the space formed between the first glass substrate 214 and the second glass substrate 213, and then, the first glass substrate 214 and the second glass substrate 213 are adhered and connected with each other by inserting the sealing member 217 into the space in the deaerating state. Accordingly, it becomes possible to prevent the moisture included in the air from influencing the scintillation layer 211, etc., resulting in increase of an operation life of the scintillation layer 211, etc., longer than ever.

In addition to the above, buffering members 218 are attached to the four corners of the detection panel 21 and to each of the intermediate positions located between any two of the four corners, respectively, in order to protect the detection panel 21 from a mechanical shock, etc., to be possibly applied from outside.

Next, an electronic circuit configuration of the detection panel 21 will be detailed in the following. Fig.16 shows an equivalent electronic circuit diagram indicating a photoelectric converting section in regard to one of pixels constituting the signal detecting section 151.

As shown in Fig. 16, the photoelectric converting section for the single pixel is constituted by a photodiode 152, and a thin film transistor 153 (hereinafter, referred to as a TFT 153, for simplicity) to take out an electric energy stored in the photodiode 152 by its switching action. The photodiode 152 serves as an image capturing element to generate an electric charge and to store the electric charge therein. The circuit is so constituted that an amplifier 154 amplifies the electric signal, taken out from the photodiode 152, to such a level that the signal readout circuit 17 can sufficiently detect the electric signal concerned.

Concretely speaking, receiving the irradiated light, the photodiode 152 generates an electric charge corresponding to an intensity of the received light. Successively, at the time when a readout voltage is applied to a gate G of the TFT 153, the electric charge flows through a channel of the TFT 153 from the photodiode 152, which is coupled to a source S of the TFT 153, to a drain D of the TFT 153, so that the electric charge is stored into a capacitor 154a coupled in parallel to the amplifier 154. Still successively, the amplifier 154 outputs an electric signal amplified in proportion to the electric charge stored in the capacitor 154a.

Still further, at the time when the amplified electric signal is outputted and taken out from the amplifier 154, a switch 154b, coupled in parallel to the amplifier 154 and the capacitor 154a, is turned ON, so as to release the electric charge stored in the capacitor 154a to reset the amplifier 154. In this connection, it is applicable that the photodiode 152 is either such a simple photodiode that has a restricted capacitance, or such a modified photodiode that includes an additional capacitor added in parallel so as to improve a dynamic range of the photo-

electric converting section.

Fig. 17 shows an equivalent electronic circuit diagram indicating a configuration in which a plurality ofphotoelectric converting sections, each of which is equivalent to the abovementioned photoelectric converting section for the single pixel, are aligned in a two dimensional pattern, and, scanning lines Ll and signal lines Lr are arranged at intervals between the rows of pixels, in such a manner that the scanning lines Ll and the signal lines Lr intersect with each other. The sources S of the TFTs 153 are coupled to the electrodes of the photodiodes 152, while the drains D of the TFTs 153 are coupled to the signal lines Lr, respectively as shown in Fig. 17. On the other hand, the other electrodes of the photodiodes 152 are coupled to each other through each of common bias lines Lb, which are further coupled to a bias voltage source 155, respectively for every line (row) of pixels as shown in Fig. 17.

The bias voltage source 155 is coupled to the controlling section 27 so as to output a bias voltage to be applied to the photodiodes 152 through the common bias lines Lb, based on an instruction signal sent from the controlling section 27. Further, the gates G of the TFTs 153 are coupled to each of the scanning lines L1, which are coupled to the scanning driving circuit 16 to be controlled by the controlling section 27. In the same way, the drains D of the TFTs 153 are coupled to each of the signal lines Lr, which are coupled to the signal readout circuit 17 to be controlled by the controlling section 27.

The signal readout circuit 17 is provided with the amplifiers 154, aforementioned, to which the signal lines Lr are coupled, respectively. At the time of the signal readout operation, the signal readout voltage is outputted onto the selected one of the scanning lines Ll, so as to apply the signal readout voltage to the gates G of the TFTs 153 coupled to the selected scanning line concerned, and as a result, the charges generated in the photodiodes 152 flow into the signal lines Lr through the TFTs 153 from the photodiodes 152, respectively. Successively, the amplifiers 154 amplify the charges transferred from the photodiodes 152, respectively, so as to take out the information stored in the photodiodes 152 for one scanning line. Still successively, with respect to all of the scanning lines Ll, the abovementioned operation is repeated for every scanning line by sequentially changing the scanning line Ll one by one, so as to take out the information stored in all of the photodiodes 152 residing within the signal detecting section 151.

Sample and hold circuits 156 are coupled to the output terminals of the amplifiers 154, respectively. The sample and hold circuits 156 are coupled to an analogue multiplexer 157 provided in the signal readout circuit 17, so that the electric signals, read out by the signal readout circuit 17, are outputted to the controlling section 27 from the analogue multiplexer 157 through an analogue to digital converter 158.

In this connection, it is applicable that the TFT 153 is categorized in either an inorganic semiconductor type, which is to be employed for an LCD (Liquid Crystal Display), or an organic semiconductor type. Further, although the photodiode 152, serving as the photoelectronic conversion element, is exemplified as the image capturing element in the present embodiment, it is also applicable that a solid-state image capturing element, other than the photodiode, is employed as the photoelectronic conversion element.

At the side sections of the signal detecting section 151, the scanning driving circuit 16 that supplies pulses to the photodiodes 152 (photoelectronic conversion elements), so as to scan and drive the photodiodes 152, and the signal readout circuit 17 to read out the electric energies stored in the photoelectronic conversion elements, respectively, are disposed.

Incidentally, the housing main section 31 is formed by employing plate-shaped carbon fibers. In the present embodiment, a sheet of plate-shaped carbon fibers 31 A, a thickness of which is in a range of 1 - 2 mm, as shown in Fig. 18, is rolled around a mold (not shown in the drawings), so as to bake it to a solid state plate under the environment of a high temperature and a high pressure, and to join an end portion 31p and another end portion 31 q in its longitudinal direction with each other. Through the abovementioned processes, the housing main section 31, shaped in a rectangular hollow cylinder and having the opening 311 and opening 312 at both of the end sections, is formed as shown in Fig. 2.

Further, when the longitudinal direction of the plate-shaped carbon fibers 31A, namely, the direction indicated by arrow S shown in Fig. 18, is defined as the rolling direction S of the plate-shaped carbon fibers 31A, the plate-shaped carbon fibers 31A is cut out from the original carbon fiber plate in such a manner that an inclination direction F of the fibers included in the plate-shaped carbon fibers 31 A versus the rolling direction S of the plate-shaped carbon fibers 31 A is set at an angle θ, other than 0°, 90°, 180° (90° x n (n = 0, 1, 2)).

In this connection, the angle θ is set at 45° in the present embodiment. Further, when the plate-shaped carbon fibers 31A is formed by laminating a plurality of plate-shaped carbon fibers plates, at least an inclination direction F of fibers included in the plate-shaped carbon fibers plate laminated as an outer-most layer of the rolled plate-shaped carbon fibers 31A is set at an angle θ, other than the abovementioned specific angles.

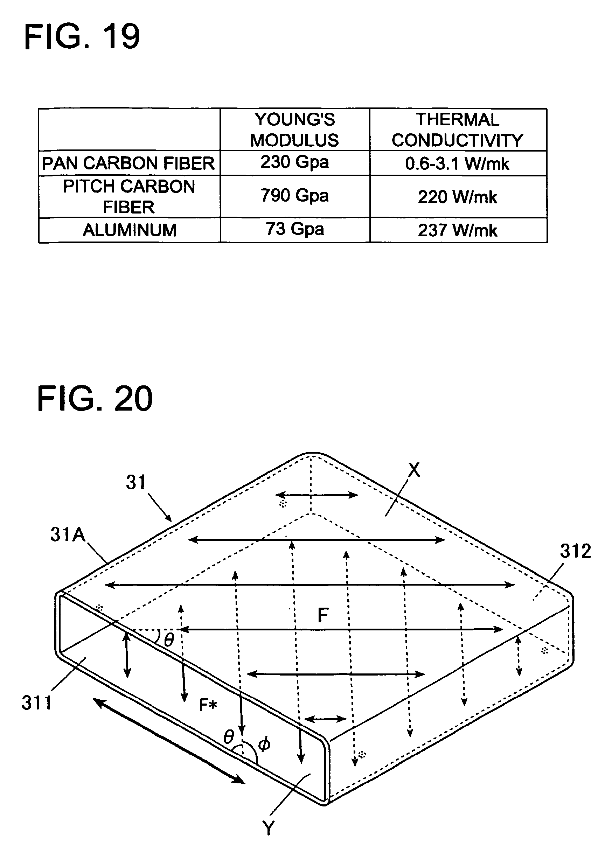

Further, it is preferable that the PITCH carbon fibers are employed for forming the housing main section 31. Fig. 19 shows a table indicating elasticity and thermal conductivity of each of the three exemplified materials for comparison purpose. Generally speaking, either the PITCH carbon fibers or the PAN (Polyacrylonitrile) carbon fibers is available as the carbon fibers. As shown in Fig. 19, however, since the elasticity of the PITCH carbon fibers is more than three times of that of the PAN carbon fibers, it is possible to acquire a sufficient strength of the housing main section 31 made of the PITCH carbon fibers, even if the plate thickness of the housing main section 31 is made to be thin.

Still further, since the thermal conductivity of the carbon fibers is generally low compared to a metal material, such as an aluminum, etc., there has bee such a problem that, if the heat is generated in the housing main section 31 made of the carbon fibers, the generated heat is not radiated to the outside of the housing, but kept inside the housing as it is. With respect to the abovementioned problem, since the PITCH carbon fibers has a high thermal conductivity being equivalent to that of the aluminum, even if various kinds of heat generating parts, such as the electric parts 22 detailed later, the rechargeable battery 25, etc., are provided inside the housing 3, it is possible to efficiently radiate the generated heat towards outside, and accordingly, it becomes possible to prevent the various kinds of parts residing inside the housing 3 from receiving negative influence caused by the accumulated heat kept therein.

Next, the operations of the cassette type detector 1, embodied in the present invention, will be detailed in the following.

As aforementioned, with respect to the first covering member 32 and the second covering member 33 (refer to Fig. 2) of the cassette type detector 1, the insertion section 322 of the first covering member 32 is inserted into the opening 311 located at one end portion of the housing main section 31, while the insertion section 332 of the second covering member 33 is inserted into the opening 312 located at the other end portion of the housing main section 31, and further, the engagement protrusions 325 and 335 are engaged with the engagement dent sections 315 and 316 of the housing main section 31, respectively.

Then, after that, the first covering member 32 and the second covering member 33 are firmly fixed onto the housing main section 31 to such an extent that both of them cannot be removed from the housing main section 31. Accordingly, all of the sections constituting the housing 3, including the housing main section 31, the first covering member 32 and the second covering member 33, are integrated into a single body.

Further, as shown in Fig. 18, the housing main section 31 is formed in the shape of rectangular hollow cylinder, having the opening 311 and the opening 312 located at both ends thereof, by rolling the plate-shaped carbon fibers 31A, in which the inclination direction F of carbon fibers versus the rolling direction S is set at an angle θ, other than 90° x n (n = 0, 1, 2).

According to abovementioned structure, as shown in Fig. 20, angle θ of the inclination direction F of the carbon fibers versus the rolling direction S of the plate-shaped carbon fibers 31A constituting the housing main section 31 is set at, for instance, 45° on a radiation incident surface X of the housing main section 31, while, on an opposite surface Y being the other side of the housing main section 31, angle φ of the inclination direction F* of the carbon fibers versus the rolling direction S becomes, for instance, 135°, which is the supplementary angle of the angle θ abovementioned (namely, in relation of θ + φ =180°).

As abovementioned, in the cassette type detector 1 embodied in the present invention, the housing main section 31 is formed in such a manner that the directions F and F* of the carbon fibers, which form the radiation incident surface X and the opposite surface Y, are inclined in respect to the opening 311 and the opening 312, respectively, and are different from each other.

Further, in the present embodiment, by rolling a sheet of the plate-shaped carbon fibers 31A, in which the inclination direction F of carbon fibers versus the rolling direction S is set at the angle θ, other than 90° × n (n = 0, 1, 2), the angle θ of the inclination direction F of the carbon fibers versus the rolling direction S on the radiation incident surface X of the housing main section 31, and the angle φ of the inclination direction F* of the carbon fibers versus the rolling direction S on the opposite surface Y, being the other side of the housing main section 31, are made to be in relation of the supplementary angle relative to each other, are made to be different from each other. Accordingly, it becomes possible to easily form the housing main section 31 so that the directions F and F* of the carbon fibers, which form the radiation incident surface X and the opposite surface Y, respectively, made to be different from each other.

In this connection, in order to prevent the detection unit 2, accommodated inside the housing 3, from being influenced by the load to be applied from the outside (such as a body weight of the patient, etc.), it is necessary to regulate the amount of bending deformation of the cassette type detector 1 shown in Fig. 26a, as a whole, within a range of the allowable bending deformation of the detection unit 2.

Accordingly, when the housing main section 31 is formed as abovementioned and the cassette type detector 1 is formed by firmly fixing the first covering member 32 and second covering member 33 thereto, the maximum bending deformation of the housing 3 of the cassette type detector 1, which is to be estimated at the time of the actual radiation-image capturing operation of the patient, and the stress to be generated in the second glass substrate 213 and the first glass substrate 214, both of which constitute the detection unit 2, will be detailed in the following, while referring to data concerned.

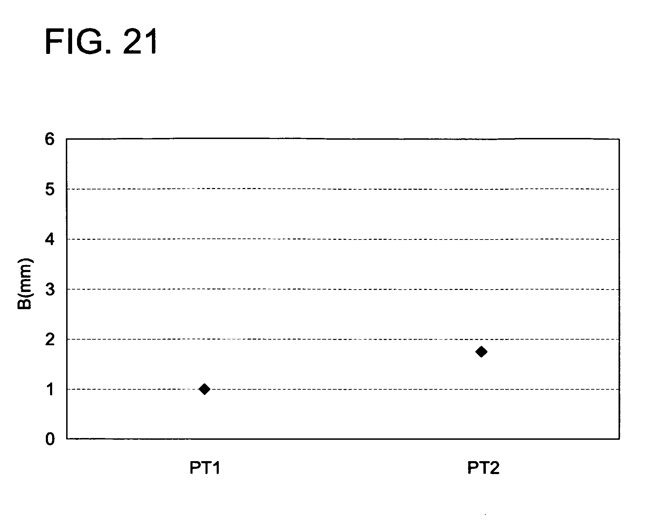

Fig. 21 shows a graph indicating simulation results in regard to the bending deformation of the housing 3 of the cassette type detector 1.

In this simulation, there employed such the housing main section 31 that is formed in the shape of rectangular hollow cylinder by employing the plate-shaped carbon fibers 31A made of PITCH carbon fibers, having a tensile modulus of elasticity of 790 Gpa, and the height of the side surface portion of the housing 3 is 16 mm, and the thickness of the housing main section 31, namely, the plate thickness of plate-shaped carbon fibers 31A, is 2 mm. Further, the size of the housing main section 31 is set at the half cut size (14 inch × 17 inch) at which the bending deformation is most liable to occur.

In this connection, the load to be applied onto the cassette type detector 1 varies depending on the image capturing posture of the subject. Within an imaginable usage environment of the cassette type detector 1, the image capturing posture of the subject, which exerts a maximum load onto the cassette type detector 1, would occur in such a case that the patient lies on his side in a recumbent position on a bed and the cassette type detector 1 is placed between the buttock of the patient and the bed (refer to Fig. 10). The simulation shown in Fig. 21 has been performed in respect to such the case that the cassette type detector 1, placed below the patient, is to be moved, while the patient lies on the bed with the abovementioned posture.

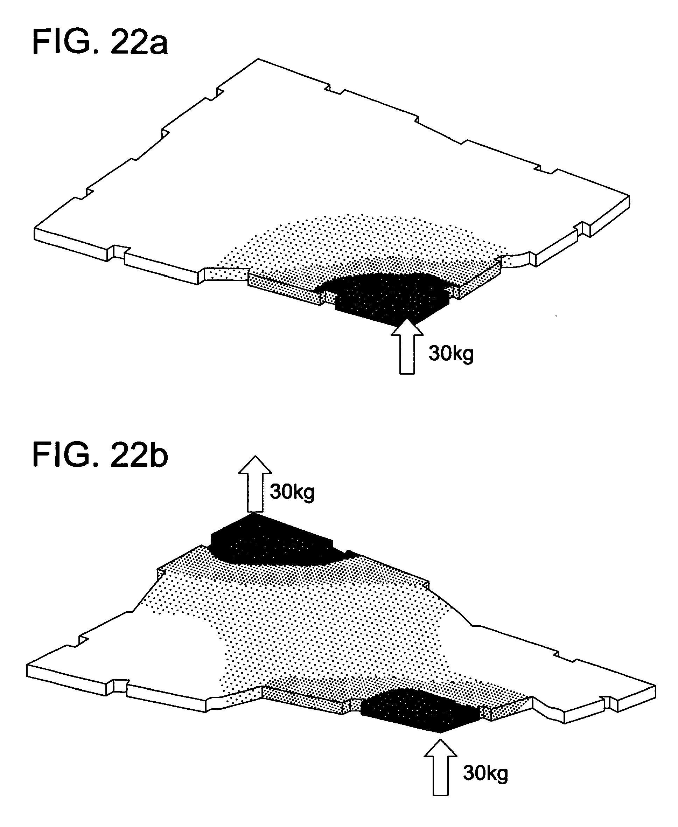

In the graph shown in Fig. 21, a pattern 1 (referred to as a PT1) represents the maximum bending deformation of the cassette type detector 1, when the cassette type detector 1 is moved in such a state that the load is applied onto the cassette type detector 1 from the upper direction, while only one end portion of the cassette type detector 1 is supported in a manner like cantilever as shown in Fig 22a. For instance, there is assumed such the case that the cassette type detector 1 is set at a position under the buttock of the patient who lies on his side in the recumbent position on the bed, and then, a single person moves the cassette type detector 1 so as to change the detecting position.

Further, a pattern 2 (referred to as a PT2) represents the other maximum bending deformation of the cassette type detector 1, when the cassette type detector 1 is moved in such a state that the load is applied onto the cassette type detector 1 in the same way as the pattern 1 (referred to as a PT1), while two ends of the cassette type detector 1, which are respectively positioned at the opposite angles, are supported as shown in Fig 22b. For instance, there is assumed such the other case that two persons move the cassette type detector 1 so as to change the detecting position.

Since the actual measuring result, such that the maximum load to be applied onto the cassette type detector 1 is 30 Kg when the cassette type detector 1 inserted below the patient who lies on his side in the recumbent position on the bed, was acquired, with respect to both the pattern 1 (referred to as a PT1) and the pattern 2 (referred to as a PT2), the amount of bending deformation (B) in such a state that the load weight of 30 kg is applied onto the end portion, which supports the cassette type detector 1, was measured

As a result of the abovementioned measurement, with respect to each of both cases abovementioned, it has been revealed that the maximum bending deformation of the housing 3 of the cassette type detector 1 can be suppressed to a value lower than 2 mm.

On the other hand, as aforementioned, when the cassette type detector 1 is formed according to the dimensional sizes in conformity with the JIS or the IEC standard, the cassette type detector 1 would be formed in a thin plate shape as a whole. Accordingly, not only the bending deformation as shown in Fig. 26a, but also the twisting deformation as shown in Fig. 26b, are liable to occur.

Further, the strongly twisted deformation is generated on the cassette type detector 1, at the time when the cassette type detector 1, which has been once set at the position under the buttock of the patient who lies on his side in a recumbent position on a bed, is to be moved in order to change the position of the cassette type detector 1, mainly like as in the case of either the pattern 1 (referred to as a PT1) or the pattern 2 (referred to as a PT2).

However, in the present embodiment, as shown in Fig. 20, the directions F and F*, being the inclination directions of the carbon fibers of the housing main section 31, are different from each other, and the inclination angles of the directions F and F* versus the rolling direction S are set at the angle θ and the supplemental angle φ, each other than 0°, 90° etc., in the radiation incident surface X and the opposite surface Y, respectively.

Due to the abovementioned structure, even if the twisting force is applied onto the cassette type detector 1, as aforementioned, and as a result, the strong tension is generated in a diagonal direction with respect to the rolling direction S, the carbon fibers, inclined at the angle θ and the supplemental angle φ, with respect to the rolling direction S in the radiation incident surface X and the opposite surface Y, respectively, impede an elongation in the direction of tension currently generated.

As described in the foregoing, according to the cassette type detector 1 embodied in the present invention, the housing main section 31 is formed by employing the plate-shaped carbon fibers 31A, and the directions F and F* of the carbon fibers of plate-shaped carbon fibers 31A, which forms both the radiation incident surface X and the opposite surface Y of the housing main section 31, are inclined with respect to the opening 311 and opening 312, respectively, and further, the housing main section 31 is formed in such a manner that the directions F and F* of the carbon fibers of the plate-shaped carbon fibers 31A, which corresponds to the radiation incident surface X and the opposite surface Y, respectively, are different from each other.

Further, concretely speaking, the housing main section 31 of the housing 3 is formed by rolling the plate-shaped carbon fibers 31A onto the mold, and by employing such the plate-shaped carbon fibers 31 A in which the directions F of the carbon fibers included therein is set at angle θ, other than 90° × n (n = 0, 1, 2), versus the rolling direction S, the directions F and F* of the carbon fibers of plate-shaped carbon fibers 31A, which forms both the radiation incident surface X and the opposite surface Y of the housing main section 31, are inclined with respect to the opening 311 and opening 312, respectively, and further, the housing main section 31 is formed in such a manner that the directions F and F* of the carbon fibers of the plate-shaped carbon fibers 31A, which corresponds to the radiation incident surface X and the opposite surface Y, respectively, are different from each other.

In this connection, when the cassette type detector 1 is formed according to the dimensional sizes in conformity with the JIS or the IEC standard, so as to make the cassette type detector 1 compatible with the CR use cassette formed in a thin type, since the cassette type detector 1 would be formed in a thin plate shape as a whole, not only the bending deformation as shown in Fig. 26a, but also the twisting deformation as shown in Fig. 26b, are liable to occur, compared to such a case that the housing 3 of the cassette type detector 1 is considerably thick. However, according to the abovementioned structure embodied in the present invention, it becomes possible to sufficiently and appropriately suppress not only the bending deformation of the housing 3, but also the twisting deformation to be generated by the tension applied in an inclined direction with respect to both the long side and the short side of the housing 3, resulting in improvement of the structural strength as a whole.

Accordingly, it becomes possible to make the cassette type detector 1, serving as such the FPD that is capable of achieving the digitalization of the image data, not only to be shaped in a thin type detector that is compatible with the CR use cassette, but also have a structural strength sufficient for preventing the housing from being deformed by the external force to be applied to the detector. In addition, the cassette type detector 1 as abovementioned is portable so as to make it possible to conduct a portable image capturing operation.

In this connection, although, as aforementioned, angle θ of the direction F of the plate-shaped carbon fibers 31 A (refer to Fig. 18 and Fig. 20) constituting the housing main section 31, versus the rolling direction S, is set at 45° in the present embodiment described in the foregoing, it is also possible that angle θ is set at 135°, while angle φ is set at 45°, being the supplemental angle of the angle θ.

As abovementioned, when angle θ of the direction F versus the rolling direction S is set at either 45° or 135°, the corresponding supplemental angle is either 135° or 45°. Therefore, as shown in Fig. 20, it becomes possible to form the housing main section 31 in such a manner that the direction F of the carbon fibers, included in the plate-shaped carbon fibers 31A within the radiation incident surface X of the housing main section 31, is orthogonal to the direction F* of the other carbon fibers, included in the plate-shaped carbon fibers 31 A within the opposite surface Y of the housing main section 31.

Accordingly, it becomes possible to further improve the strength of the housing 3 against the twisting force to be applied in an inclined direction with respect to both the long side and the short side of the housing 3 of the cassette type detector 1.

Further, it is also possible to set the angle θ of the direction F of the plate-shaped carbon fibers 31 A (refer to Fig. 18 and Fig. 20) constituting the housing main section 31, versus the rolling direction S, at an angle, other than 45° employed in the present embodiment described in the foregoing.

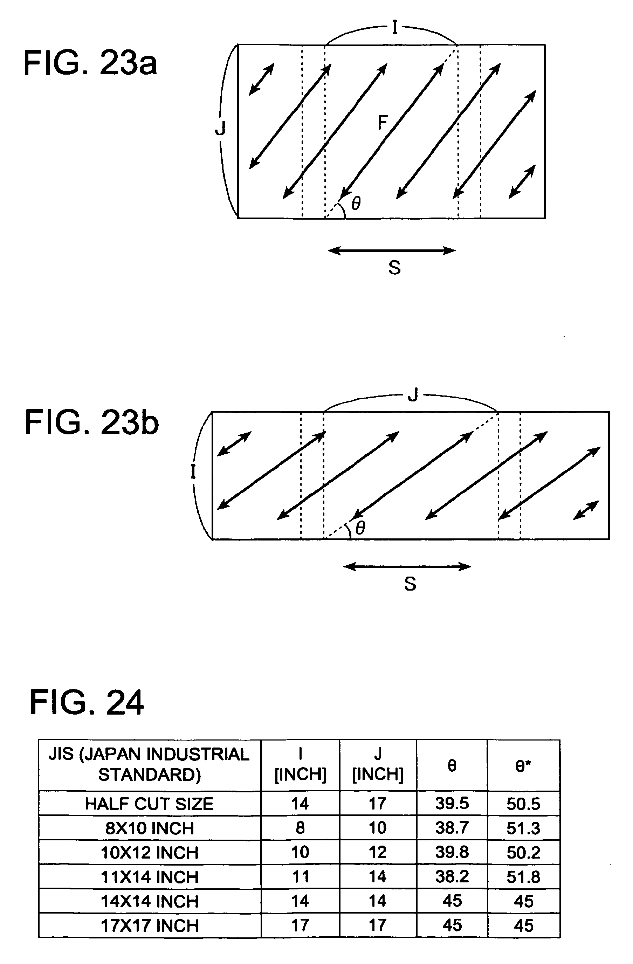

For instance, describing such a case that it is taken into account the fact that the tension generated by the twisting force applied to the housing 3 is mainly exerted in the diagonal direction of its rectangular shape, even if the housing 3 is formed in the same rectangular shape, either the case that the rolling direction S of the plate-shaped carbon fibers 31A is established as the extended direction of the short side I as shown in Fig. 23a, or the other case that the rolling direction S is established as the extended direction of the long side J as shown in Fig. 23b, is applicable.

In the abovementioned situation, the angle θ of the direction F of the plate-shaped carbon fibers 31A versus the rolling direction S, in the case shown in Fig. 23a, and the other angle θ in the other case shown in Fig. 23b, are complementary relative to each other, namely, their sum is 90°. Further, when the other angle θ in the other case shown in Fig. 23b is defined as angle θ*, angles θ and angles θ* applicable for the dimensional sizes in conformity with the JIS or the IEC standard, for instance, are indicated in the table shown in Fig. 24.

As described in the above, instead of setting angle θ of the direction F of the plate-shaped carbon fibers 31A constituting the housing main section 31, versus the rolling direction S, at 45°, as described in the aforementioned embodiment, it is possible to set angle θ at an arbitral angle within a range of 30° - 60° or its supplemental angle within a range of 120° - 150° so as to direct the carbon fibers towards the diagonal direction of the rectangular shape, and angle θ may be set at a suitable value by considering the intensity of the housing 3, as needed.

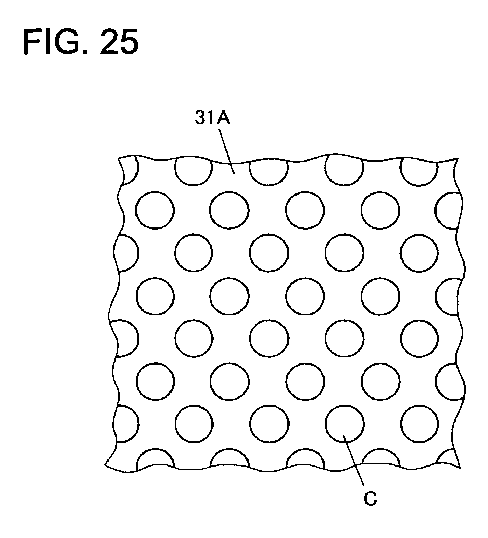

Further, it is preferable that an irregularity C, for instance, a patterned indented surface as shown in Fig. 25, is formed on the surface of the plate-shaped carbon fibers 31A. When the housing 3 of the cassette type detector 1 is formed in such a manner that its thickness in the radiation incident direction is set at a value being equal to or smaller that 16 mm so as to make its dimensional sizes comply with the JIS or IEC standard in regard to the CR use cassette or the conventional screen/film use cassette, it is preferable that the irregularity C is formed in such a manner that protrusions are protruded towards the inside of the housing 3.

According to the simulation experiments, conducted by the present inventors, in which the housing main section 31 is formed by employing such the plate-shaped carbon fibers 31A, thickness of which is 2 mm and onto which concavities and convexities, each diameter being 6 mm and each depth being 0.5 mm, are formed as shown in Fig. 25, so as to investigate the bending deformation of the housing 3 of the cassette type detector 1 formed by employing the plate-shaped carbon fibers 31A abovementioned, it has been revealed that the amount of the bending deformation of the housing 3 could be alleviated (reduced) by around 10 %, compared to the case that the cassette type detector 1 is formed without forming the concavities and convexities as abovementioned.

Although it is needless to say that the amount of the bending deformation of the housing 3 could be reduced by making the thickness of the plate-shaped carbon fibers 31A thicker than ever, the thicker the thickness of the plate-shaped carbon fibers 31A is set, the heavier the weight of the cassette type detector 1 increase. Further, by making the thickness of the plate-shaped carbon fibers 31A thicker than ever, it becomes easy for the housing 3 and the detection unit 2 incorporated therein to mechanically contact each other, and as a result, the design flexibility of the detection unit 2 is made to be restricted.

On the other hand, as aforementioned, by forming the irregularity C onto the surface of the plate-shaped carbon fibers 31A constituting the housing main section 31, it becomes possible to reduce the amount of the bending deformation of the cassette type detector 1 without making the thickness of the plate-shaped carbon fibers 31A thicker than ever, and accordingly, without generating the abovementioned problems.

**Claims**

1. Cassette-type radiation image detector (1), comprising

   - a detection unit (2) to detect incident radiation so as to generate radiation image data; and
   - a housing (3) that includes a main section (31) formed in shape of a rectangular hollow cylinder, at both ends of which opening sections (311; 312) are provided, by employing plate-shaped carbon fibers (31 A) and a first covering member (32) and a second covering member (33), each of which is engaged with the main section by using an engaging member, so as to cover each of the opening sections, and that accommodates the detection unit therein;

   **characterized in that** the main section (31) is formed in such a manner that a first direction (F) of carbon fibers included in a radiation incident surface (X), serving as a surface of the main section, and a second direction (F*) of carbon fibers, included in an opposite surface (Y), serving as another surface of the main section, are Inclined relative to the opening sections (311; 312), respectively, and are different from each other.

2. Radiation image detector recited in claim 1, **characterized in that** the main section (31) is formed by employing plate-shaped carbon fibers (31 A) and rolling the plate-shaped carbon fibers, and the angle of the first direction (F) of the carbon fibers versus the rolling direction (S) of the plate-shaped carbon fibers (31A) to be rolled is in the range of 30° to 60° or in the range of 120° to - 150°.

3. Radiation image detector recited in claim 1 or 2, **characterized in that** the main section (31) is formed by employing the plate-shaped carbon fibers (31A) and rolling the plate-shaped carbon fibers, and the angle of the first direction (F) of the carbon fibers versus the rolling direction of the plate-shaped carbon fibers (31A) to be rolled is 45° or 135°.

4. Radiation image detector recited in any one of claims 1 to 3, **characterized in that** an irregularity (C) is formed on the surface of the plate-shaped carbon fibers (31 A).

5. Radiation image detector recited in any one of claims 1 to 4, **characterized in that** the outer dimensional sizes of the housing (3) are in conformity with those specified by IEC 60406.

6. Radiation image detector recited in any one of claims 1 to 5, **characterized in that** the thickness of the housing (3) in radiation incident direction is equal to or smaller than 16 mm.

7. Radiation image detector recited in any one of claims 1 to 6, **characterized in that** the detection unit (2) includes a scintillator (211) to convert the incident radiation to light and a detecting section (151) that receives the light converted by the scintillator and further converts this light to electric signals.

**Patentansprüche**

1. Kassettenförmiger Strahlungsbilddetektor (1) umfassend

    - eine Nachweiseinheit (2) zum Nachweis von einfallender Strahlung, so dass Strahlungsbilddaten erzeugt werden; und
    - ein Gehäuse (3), das einen in Form eines rechteckigen Hohlzylinders ausgebildeten Hauptabschnitt (31) umfasst, an dessen beiden Enden Öffnungsteile (311; 312) vorgesehen sind, durch Verwendung von plattenförmigen Kohlenfasern (31A) und eines ersten Abdeckelements (32) und eines zweiten Abdeckelements (33), wovon jedes in den Hauptabschnitt unter Verwendung eines Eingriffselements eingreift, so dass die Öffnungsabschnitte jeweils bedeckt sind, und das die Nachweiseinheit darin aufnimmt;

**dadurch gekennzeichnet, dass** der Hauptabschnitt (31) so ausgebildet ist, dass eine erste Richtung (F) von in einer Strahlungseinfallfläche (X) eingeschlossenen Kohlenfasern, die als eine Oberfläche des Hauptabschnitts dient, und eine zweite Richtung (F*) von in einer gegenüberliegenden Fläche (Y) eingeschlossenen Kohlenfasern, die als eine andere Oberfläche des Hauptabschnitts dient, bezüglich der Öffnungsabschnitte (311 ; 312) geneigt und voneinander verschieden sind.

2. Kassettenförmiger Strahlungsbilddetektor nach Anspruch 1,
**dadurch gekennzeichnet, dass** der Hauptabschnitt (31) unter Verwendung der plattenförmigen Kohlefasern (31A) und Rollen der plattenförmigen Kohlenfasern ausgebildet ist, und der Winkel der ersten Richtung (F) der Kohlenfaser bezüglich der Rollrichtung (S) der zu rollenden plattenförmigen Kohlefasern (31 A) im Bereich von 30° bis 60° oder im Bereich von 120° bis 150° liegt.

3. Kassettenförmiger Strahlungsbilddetektor nach Anspruch 1 oder 2,
**dadurch gekennzeichnet, dass** der Hauptabschnitt (31) unter Verwendung der plattenförmigen Kohlefasern (31 A) und Rollen der plattenförmigen Kohlenfasern ausgebildet ist, und der Winkel der ersten Richtung (F) der Kohlenfaser bezüglich der Rollrichtung der zu rollenden plattenförmigen Kohlefasern (31A) 45° oder 135° beträgt.

4. Kassettenförmiger Strahlungsbilddetektor nach einem der Ansprüche 1 bis 3,
**dadurch gekennzeichnet, dass** eine Unregelmäßigkeit (C) auf der Oberfläche der plattenförmigen Kohlefasern (31 A) ausgebildet ist.

5. Kassettenförmiger Strahlungsbilddetektor nach einem der Ansprüche 1 bis 4
**dadurch gekennzeichnet, dass** die äußeren Größendimensionen des Gehäuses (3) konform sind zu denjenigen, die durch IEC60406 spezifiziert sind.

6. Kassettenförmiger Strahlungsbilddetektor nach einem der Ansprüche 1 bis 5,
**dadurch gekennzeichnet, dass** die Dicke des Gehäuses (3) in Richtung einfallender Strahlung gleich oder kleiner ist als 16 mm.

7. Kassettenförmiger Strahlungsbilddetektor nach einem der Ansprüche 1 bis 6,
**dadurch gekennzeichnet, dass** die Nachweiseinheit (2) einen Szintillator (211) umfasst, um die einfallende Strahlung in Licht umzuwandeln und ein Nachweisabschnitt (151), der das durch den Szintillator umgewandelte Licht aufnimmt, dieses Licht weiter in elektrische Signale umwandelt.

**Revendications**

1. Détecteur d'image de rayonnement de type cassette (1) comprenant

   - une unité de détection (2) pour la détection de rayonnement incident afin de générer des données d'image de rayonnement ; et
   - un boîtier (3) qui comprend une partie principale (31) formée en forme d'un cylindre creux rectangulaire, aux deux extrémités duquel des parties d'ouvertures (311; 312) sont prévues en utilisant des fibres de carbone en forme de plaque (31A) et un premier membre de couverture (32) et un deuxième membre de couverture (33), dont chacun est engagé avec la partie principale en utilisant un membre d'engagement afin de couvrir chacun des parties d'ouverture, et qui y reçoit l'unité de détection ;

   **caractérisé par le fait que** la partie principale (31) est formée d'une telle manière que une première direction (F) des fibres de carbone inclus dans une surface d'incidence de rayonnement (X) servant en tant qu'une surface de la partie principale, et une deuxième direction (F*) de fibres de carbone inclus dans une surface opposée (Y), servant en tant qu'une autre surface de la partie principale, sont inclinées en relation aux parties d'ouverture (311 ; 312), respectivement, et sont différentes l'une de l'autre.

2. Détecteur d'image de rayonnement de type cassette selon la revendication 1, **caractérisé par le fait que** la partie principale (31) est formée en utilisant des fibres de carbone en forme de plaque (31A) et roulant les fibres de carbone en forme de plaque, et l'angle de la première direction (F) des fibres de carbone par rapport à la direction de roulement (S) des fibres de carbone en forme de plaque (31A) à rouler est dans la plage de 30° à 60° ou dans la plage de 120° à 150°.

3. Détecteur d'image de rayonnement de type cassette selon la revendication 1 ou 2, **caractérisé par le fait que** la partie principale (31) est formée en utilisant les fibres de carbone en forme de plaque (31A) et roulant les fibres de carbone en forme de plaque, et l'angle de la première direction (F) des fibres de carbone par rapport à la direction roulant des fibres de carbone en forme de plaque (31A) à rouler est 45° ou 135°.

4. Détecteur d'image de rayonnement de type cassette selon l'une des revendications 1 à 3, **caractérisé par le fait qu'**une irrégularité (C) est formée sur la surface des fibres de carbone en forme de plaque (31A).

5. Détecteur d'image de rayonnement de type cassette selon l'une des revendications 1 à 4, **caractérisé par le fait que** les tailles dimensionnelles extérieures du boîtier (3) sont en conformité avec celles spécifiées par IEC60406.

6. Détecteur d'image de rayonnement de type cassette selon l'une des revendications 1 à 5, **caractérisé par le fait que** l'épaisseur du boîtier (3) en direction de rayonnement incident est égale ou inférieur à 16 mm.

7. Détecteur d'image de rayonnement de type cassette selon l'une des revendications 1 à 6, **caractérisé par le fait que** l'unité de détection (2) comprend un scintillateur (211) afin de convertir le rayonnement incident en lumière et une partie de détection (151) qui reçoit la lumière convertie par le scintillateur et convertit en outre cette lumière en signaux électriques.

# FIG. 1

# FIG. 2

# FIG. 3a

333

2

# FIG. 3b

333

2

# FIG. 3c

333

2

## FIG. 4

## FIG. 5

# FIG. 6

# FIG. 7

# FIG. 8

# FIG. 9

# FIG. 10

# FIG. 11

# FIG. 12

UNIT: MPa(kgf/cm$^2$)

| KIND OF GLASS | NOMINAL THICKNESS | SHORT TIME ALLOWABLE STRESS | | LONG TERM ALLOWABLE STRESS | |
|---|---|---|---|---|---|
| | | WITHIN SURFACE ($\sigma$ ac) | EDGE PORTION ($\sigma$ ae) | WITHIN SURFACE ($\sigma$ ac) | EDGE PORTION ($\sigma$ ae) |
| FLOAT FLAT GLASS. HEAT RAYS ABSORPTION FLAT GLASS. HEAT RAYS REFLECTION FLAT GLASS. | EQUAL TO OR SMALLER THAN 8 mm | 24.5 (250) | 17.7 (180) | 9.8 (100) | 6.9 (70) |
| | GREATER THAN 8 mm AND EQUAL TO OR SMALLER THAN 12 mm | 22.1 (225) | 17.7 (180) | 8.8 (90) | 6.9 (70) |
| | GREATER THAN 12 mm AND EQUAL TO OR SMALLER THAN 20 mm | 19.6 (200) | 17.7 (180) | 7.8 (80) | 6.9 (70) |
| | GREATER THAN 20 mm | 18.6 (190) | 17.7 (180) | 7.4 (75) | 6.9 (70) |
| WIRE-WIRE POLISHED GLASS | | 19.6 (200) | 9.8 (100) | 7.8 (80) | 3.9 (40) |
| WIRE-WIRE FIGURED GLASS | | 14.7 (150) | 9.8 (100) | 5.9 (60) | 39 (40) |
| TEMPERED GLASS | | 88.3 (900) | 79.4 (810) | 73.5 (750) | 68.6 (700) |
| DOUBLE TEMPERED GLASS | | 44.1 (450) | 35.3 (360) | 29.4 (300) | 24.5 (250) |

# FIG. 13

| | IN CASE OF SUPPORTING FOUR SIDES | REMARK |
|---|---|---|
| GLASS SIZE OF SIDE a mm | 364.6 | |
| GLASS SIZE OF SIDE b mm | 440.6 | |
| b/a | 1.2 | |
| $\beta 1$ | 0.362 | |
| $\alpha 1$ | 0.064 | |
| GLASS THICKNESS t mm | 1.2 | ESTABLISHED AS "0.6 mm X 2 = 1.2" |
| LOAD TO BE APPLIED ONTO GLASS kg | 11.1 | |
| LOAD TO BE APPLIED ONTO GLASS N | 108.78 | 1kg=9.8N |
| UNIFORMLY DISTRIBUTED LOAD W N/m$^2$ | 677 | |
| UNIFORMLY DISTRIBUTED LOAD W MPa | 0.00067715 | |
| YOUNG'S MODULUS OF FLAT GLASS E MPa | 71600 | |
| MAXIMUM STRESS $\sigma$ MPa | 23 | |
| MAXIMUM AMOUNT OF BENDING DEFORMATION $\delta$ mm | 6 | |
| ALLOWABLE STRESS MPa | 24.5 | |

## FIG. 14

## FIG. 15

COEFFICIENT VALUE: $\beta_1$, $\alpha_1$

| b/a | 1.0 | 1.1 | 1.2 | 1.3 | 1.4 | 1.5 | 1.6 | 1.7 | 1.8 | 1.9 | 2.0 |
|---|---|---|---|---|---|---|---|---|---|---|---|
| $\beta_1$ | 0.272 | 0.318 | 0.362 | 0.403 | 0.441 | 0.475 | 0.507 | 0.535 | 0.580 | 0.583 | 0.603 |
| $\alpha_1$ | 0.046 | 0.055 | 0.064 | 0.703 | 0.081 | 0.088 | 0.094 | 0.100 | 0.106 | 0.111 | 0.115 |

## FIG. 16

## FIG. 17

## FIG. 18

# FIG. 19

| | YOUNG'S MODULUS | THERMAL CONDUCTIVITY |
|---|---|---|
| PAN CARBON FIBER | 230 Gpa | 0.6-3.1 W/mk |
| PITCH CARBON FIBER | 790 Gpa | 220 W/mk |
| ALUMINUM | 73 Gpa | 237 W/mk |

# FIG. 20

# FIG. 21

FIG. 22a

30kg

FIG. 22b

30kg

30kg

FIG. 23a

FIG. 23b

FIG. 24

| JIS (JAPAN INDUSTRIAL STANDARD) | I [INCH] | J [INCH] | θ | θ* |
|---|---|---|---|---|
| HALF CUT SIZE | 14 | 17 | 39.5 | 50.5 |
| 8X10 INCH | 8 | 10 | 38.7 | 51.3 |
| 10X12 INCH | 10 | 12 | 39.8 | 50.2 |
| 11X14 INCH | 11 | 14 | 38.2 | 51.8 |
| 14X14 INCH | 14 | 14 | 45 | 45 |
| 17X17 INCH | 17 | 17 | 45 | 45 |

# FIG. 25

# FIG. 26a

# FIG. 26b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005121783 A
- JP 2005114944 A
- JP 9073144 A
- JP 2002031152 A
- US 7189972 B2